# EUROPEAN PATENT SPECIFICATION

(11) **EP 3 788 167 B1**
(45) Date of publication and mention of the grant of the patent: **15.11.2023**
(21) Application number: 19796415.8
(22) Date of filing: 01.05.2019
(51) Int. Cl.: C12Q 1/6886, G16B 99/00

(54) **METHODS OF DIAGNOSING AND TREATING PATIENTS WITH CUTANEOUS SQUAMOUS CELL CARCINOMA**
VERFAHREN ZUR DIAGNOSE UND BEHANDLUNG VON PATIENTEN MIT EINEM KUTANEN PLATTENEPITHELKARZINOM
PROCÉDÉS DE DIAGNOSTIC ET DE TRAITEMENT DE PATIENTS ATTEINTS D'UN CARCINOME ÉPIDERMOÏDE CUTANÉ

(30) Priority: 02.05.2018 US 201862665872 P; 27.09.2018 US 201862737863 P
(43) Date of publication of application: 10.03.2021
(73) Proprietor: Castle Biosciences, Inc., Friendswood, TX 77546 (US)
(72) Inventor: COOK, Robert Willis, Friendswood, TX 77546 (US); COVINGTON, Kyle R., Friendswood, TX 77546 (US); MAETZOLD, Derek, Friendswood, TX 77546 (US)
(74) Representative: Zwicker, Jörk
(86) International application number: PCT/US2019/030282
(87) International publication number: WO 2019/213321

(56) References cited:
- US-A1- 2011 124 525
- US-A1- 2013 224 192
- US-A1- 2015 152 474
- US-A1- 2016 058 777
- KUNZ ET AL: "DNA Microarray Technology in Dermatology", SEMINARS IN CUTANEOUS MEDICINE AND SURGERY, W.B. SAUNDERS, PHILADELPHIA, US, vol. 27, no. 1, 1 March 2008 (2008-03-01), pages 16-24, XP022646957, ISSN: 1085-5629, DOI: 10.1016/J.SDER.2007.12.004 [retrieved on 2008-05-24]
- HAMEETMAN et al.: "Molecular profiling of cutaneous squamous cell carcinomas and actinic keratoses from organ transplant recipients", BMC Cancer, vol. 13, no. 1, 5 February 2013 (2013-02-05), page 58, XP021139054, DOI: 10.1186/1471-2407-13-58

## Description

### FIELD OF THE DISCLOSURE

The present disclosure relates to methods for predicting the risk of recurrence and/or metastasis in primary cutaneous squamous cell carcinoma (cSCC).

### BACKGROUND

Cutaneous squamous cell carcinoma (cSCC) is rivaled only by basal cell carcinoma as the most common cancer in the U.S. Though most cases are cured by excision, a subset recur and become incurable with the number of deaths approximating melanoma (Karia et al., J. Am. Acad. Dermatol. 68(6): 957-66 (2013)). Despite overall good prognosis for patients with cSCC, a subset will develop local, regional, or distant recurrences following complete excision of the primary tumor. Those at high risk of recurrence are eligible for adjuvant treatment options. While specific clinical features are associated with recurrence, they collectively fail to identify 30-40% of all cSCC recurrences and many tumors that possess high risk features will not recur. Prediction models with increased positive predictive values are needed to prevent unnecessary procedures. To address the need for more accurate predictive factors and facilitate appropriate intervention strategies, gene expression analysis was used to determine a signature associated with recurrence in patients with cSCC. US 2013/224192 discloses methods for the prognosis of the progression of cancer in a patient comprising determining in a tumor tissue sample the expression level of the FERMT1 gene.

### SUMMARY

There is a need in the art for a more objective method of predicting which tumors display aggressive metastatic activity. Development of an accurate molecular footprint, such as the gene expression profile assay disclosed herein, would be a significant advance forward for the field. A multi-center study using archived primary tissue samples with extensive capture of associated clinical data (subjects with pathologically confirmed cSCC diagnosed after 2006, minimum 2 years of follow-up, and two separate outcome measures: nodal/distant metastasis and local recurrence) was used to identify gene expression profiles that accurately predict primary cSCC with a high risk of regional nodal/distant metastasis, and primary cSCC with high risk of local recurrence after complete surgical clearance.

The present invention provides a method for predicting risk of recurrence, metastasis, or both, in a patient with a cutaneous squamous cell carcinoma (cSCC) tumor, the method comprising: (a) isolating mRNA from a cSCC tumor sample that has been obtained from the patient; (b) determining the expression level of ALOX12, BBC, BHLHB9, GTPBP2, HDDC3, ID2, LCE2B, LOC100287896, MMP10, MSANTD4, NFASC, NFIC, PDPN, RCHY1, RPP38, RUNX3, TAF6L, TFAP2B, ZNF48, and ZNF496; (c) providing an indication as to whether the cSCC tumor has a low risk to a high risk of local recurrence, distant metastasis, or both, based on the expression levels of ALOX12, BBC, BHLHB9, GTPBP2, HDDC3, ID2, LCE2B, LOC100287896, MMP10, MSANTD4, NFASC, NFIC, PDPN, RCHY1, RPP38, RUNX3, TAF6L, TFAP2B, ZNF48, and ZNF496.

In some embodiments, the expression level of each gene in the gene set is determined by reverse transcribing the isolated mRNA into cDNA and measuring a level of fluorescence for each gene in the gene set by a nucleic acid sequence detection system following Real-Time Polymerase Chain Reaction (RT-PCR). In certain embodiments, the cSCC tumor sample is obtained from formalin-fixed, paraffin embedded sample.

In another embodiment, the method comprises comparing the expression levels of ALOX12, BBC, BHLHB9, GTPBP2, HDDC3, ID2, LCE2B, LOC100287896, MMP10, MSANTD4, NFASC, NFIC, PDPN, RCHY1, RPP38, RUNX3, TAF6L, TFAP2B, ZNF48, and ZNF496 from the cSCC tumor with the expression levels of ALOX12, BBC, BHLHB9, GTPBP2, HDDC3, ID2, LCE2B, LOC100287896, MMP10, MSANTD4, NFASC, NFIC, PDPN, RCHY1, RPP38, RUNX3, TAF6L, TFAP2B, ZNF48, and ZNF496 from a predictive training set to generate a probability score of the risk of local recurrence, distant metastasis, or both. In one embodiment, the probability score is a bimodal, two-class analysis, wherein a patient having a value of between 0 and 0.499 is designated as class 1 with a low risk of local recurrence, distant metastasis, or both, and a patient having a value of between 0.500 and 1.00 is designated as class 2 with an increased risk of local recurrence, distant metastasis, or both.

In certain embodiments, the expression level of: ALOX12 is decreased, BBC is increased, BHLHB9 is decreased, GTPBP2 is decreased, HDDC3 is increased, ID2 is decreased, LCE2B is decreased, LOC100287896 is increased, MMP10 is decreased, MSANTD4 is decreased, NFASC is decreased, NFIC is decreased, PDPN is increased, RCHY1 is increased, RPP38 is decreased, RUNX3 is increased, TAF6L is increased, TFAP2B is decreased, ZNF48 is increased, and ZNF496 is increased, when comparing a recurrent tumor sample to a non-recurrent tumor sample. In other embodiments, the increase or decrease in the expression level is the gene level from a metastatic tumor sample versus a non-metastatic tumor sample.

Other aspects and embodiments are disclosed in the accompanying claims and will become apparent from the following detailed description and claims, with reference, where appropriate, to the accompanying drawings.

### BRIEF DESCRIPTION OF THE DRAWINGS

**FIG. 1** shows the study design workflow.
**FIG. 2** shows the differential expression of 18 genes found to be significantly differentially expressed between recurrent (Rec) and non-recurrent (NR) cSCC cases

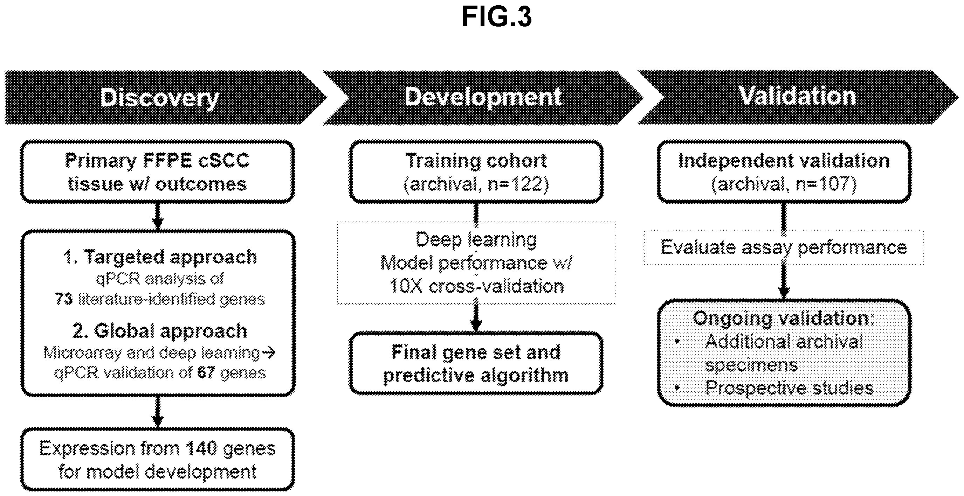

**FIG. 3** shows another exemplary study design workflow.
**FIG. 4** shows a metastasis-free survival curve for low risk, Class 1, and high-risk, Class 2, tumors using the 20-1 gene set.

### DETAILED DESCRIPTION

Despite overall good prognosis for patients with cSCC, a subset will develop local, regional, or distant recurrences following complete excision of the primary tumor. Those at high risk of recurrence are eligible for adjuvant treatment options. While specific clinical features are associated with recurrence, they collectively fail to identify 30-40% of all cSCC recurrences and many tumors that express high risk features will not recur. To address the need for more accurate predictive factors and facilitate appropriate intervention strategies, a gene expression analysis was used to determine a signature associated with recurrence in cSCC. In that analysis, 140 candidate genes were selected for evaluation of gene expression changes in recurrent and non-recurrent cases. A total of 230 primary cSCC tumors were collected under an IRB-approved, multi-center protocol and analyzed. After quality filtering, expression of the genes was assessed across 212 samples. Multiple subsets of genes were significantly differentially expressed between recurrent and non-recurrent cases. The results demonstrate that gene expression differences can distinguish between recurrent and non-recurrent cSCC. Such gene expression differences can help identify those patients who might benefit from additional therapeutic interventions and treatments.

Unless otherwise defined, all technical and scientific terms used herein have the same meaning as would be commonly understood by one of ordinary skill in the art to which the claimed invention belongs. Although methods and materials similar or equivalent to those described herein can be used to practice the methods and kits disclosed or claimed herein, suitable methods and materials are described below. In case of conflict, the present specification, including definitions, will control. In addition, the materials, methods, and examples are illustrative only and are not intended to be limiting. Other features and advantages of the claimed invention will be apparent from the following detailed description.

As used herein, the singular forms "a," "an," and "the" include plural referents unless the context clearly dictates otherwise. For example, reference to "a nucleic acid" means one or more nucleic acids.

It is noted that terms like "preferably," "commonly," and "typically" are not utilized herein to limit the scope of the claimed invention or to imply that certain features are critical, essential, or even important to the structure or function of the claimed invention. Rather, these terms are merely intended to highlight alternative or additional features that can or cannot be utilized in a particular embodiment disclosed or claimed herein.

As used herein, the terms "polynucleotide," "nucleotide," "oligonucleotide," and "nucleic acid" can be used interchangeably to refer to nucleic acid comprising DNA, cDNA, RNA, derivatives thereof, or combinations thereof.

In an embodiment, a method for predicting risk of recurrence, metastasis, or both, in a patient with a cutaneous squamous cell carcinoma (cSCC) tumor is disclosed herein, the method comprising: (a) isolating mRNA from a cSCC tumor sample that has been obtained from the patient; (b) determining the expression level of ALOX12, BBC, BHLHB9, GTPBP2, HDDC3, ID2, LCE2B, LOC100287896, MMP10, MSANTD4, NFASC, NFIC, PDPN, RCHY1, RPP38, RUNX3, TAF6L, TFAP2B, ZNF48, and ZNF496; (c) providing an indication as to whether the cSCC tumor has a low risk to a high risk of local recurrence, distant metastasis, or both, based on the expression levels of ALOX12, BBC, BHLHB9, GTPBP2, HDDC3, ID2, LCE2B, LOC100287896, MMP10, MSANTD4, NFASC, NFIC, PDPN, RCHY1, RPP38, RUNX3, TAF6L, TFAP2B, ZNF48, and ZNF496.

In some embodiments, the expression level of each gene in the gene set is determined by reverse transcribing the isolated mRNA into cDNA and measuring a level of fluorescence for each gene in the gene set by a nucleic acid sequence detection system following Real-Time Polymerase Chain Reaction (RT-PCR). In certain embodiments, the cSCC tumor sample is obtained from formalin-fixed, paraffin embedded sample.

In another embodiment, the gene set comprises the genes ACSBG1, AIM2, ALOX12, ANXA9, APOBEC3G, ARPC2, ATP6AP1, ATP6V0E2, BBC, BHLHB9, BLOC1S1, C1QL4, C21orf59, C3orf70, CCL27, CD163, CEP76, CHI3L1, CHMP2B, CXCL10, CXCR4, CYP2D6 (LOC101929829), DARS, DCT, DDAH1, DSS1, DUXAP8, EGFR, EphB2, FCHSD1, FDFT1, FLG, FN1, GTPBP2, HDDC3, HNRNPL, HOXA10 (HOXA9, MIR196B), HPGD, ID2, IL24, IL2RB, IL7R, INHBA, IPO5P1, KIT, KLK5, KRT17, KRT 18, KRT 19, KRT6B, LAMC2, LCE2B, LIME1 (ZGPAT), LOC100287896, LOC101927502, LOR, LRRC47, MIER2, MIR129-1, MIR3916, MKLN1, MMP1, MMP10, MMP 12, MMP13, MMP3, MMP7, MMP9, MRC1, MRPL21, MSANTD4, MYC, NEB, NEFL, NFASC, NFIA, NFIB, NFIC, NOA1, PD1, PDL1, PDPN, PI3, PIG3, PIGBOS1, PIM2, PLAU, PLS3, PTHLH, PTRHD1, RBM33, RCHY1, RNF135, RPL26L1, RPP38, RUNX3, S100A8, S100A9, SEPT3, SERPINB2, SERPINB4, SLC1A3, SLC25A11, SNORD124, SPATA41, SPP1, TAF6L, TFAP2B, THYN1, TMEM41B, TNNC1, TUBB3, TUFM (MIR4721), TYRP1, UGP2, USP7, VIM, YKT6, ZNF48, ZNF496, ZNF839, and/or ZSCAN31.

In an embodiment, the method further comprises comparing the expression levels of of ALOX12, BBC, BHLHB9, GTPBP2, HDDC3, ID2, LCE2B, LOC100287896, MMP10, MSANTD4, NFASC, NFIC, PDPN, RCHY1, RPP38, RUNX3, TAF6L, TFAP2B, ZNF48, and ZNF49 from the cSCC tumor sample to the expression levels of ALOX12, BBC, BHLHB9, GTPBP2, HDDC3, ID2, LCE2B, LOC100287896, MMP10, MSANTD4, NFASC, NFIC, PDPN, RCHY1, RPP38, RUNX3, TAF6L, TFAP2B, ZNF48, and ZNF49 from a predictive training set to generate a probability score of the risk of local recurrence, distant metastasis, or both. In one embodiment, the probability score is a bimodal, two-class analysis, wherein a patient having a value of between 0 and 0.499 is designated as class 1 with a low risk of local recurrence, distant metastasis, or both, and a patient having a value of between 0.500 and 1.00 is designated as class 2 with an increased risk of local recurrence, distant metastasis, or both.

As used herein, the term "recurrence" refers to the recurrence or disease progression that may occur locally (such as local recurrence and in transit disease), regionally (such as nodal micrometastasis or macrometastasis), or distally (such as brain, lung and other tissues). Risk, as used herein, includes low-risk or high-risk of metastasis according to any of the statistical methods disclosed herein. In one embodiment, risk of recurrence or metastasis for cSCC can be classified from a low risk to a high risk (for example, the cSCC tumor has a graduated risk from low risk to high risk or high risk to low risk of local recurrence, locoregional recurrence, or distant metastasis). In other embodiments, low risk refers to a 5-year relapse-free survival rate, a 5-year metastasis free survival rate, or a 5-year disease specific survival rate of greater than 50%, 55%, 60%, 65%, 70%, 75%, 80%, 85%, 90%, 95%, or more than 95%, and high risk refers to a 5-year relapse-free survival rate, a 5-year metastasis free survival rate, or a 5-year disease specific survival rate of less than 50%, 45%, 40%, 35%, 30%, 25%, 20%, 15%, 10%, 5%, or less than 5%.

In certain embodiments, risk stratifications may be binned, for example a group with an arbitrary designation Class A may be selected based on recurrence risk of less than 25%, 20%, 15%, 10%, 5%, or less than 5%. A group with arbitrary designation Class B may be selected based on a risk of 75%, 70%, 65%, 60%, 55%, 50%, 45%, 40%, 35%, 30%, 25%, or any number in between. A group with arbitrary designation Class C may be selected based on a risk of 75%, 80%, 85%, 90%, 95%, or higher than 95%. These class designations may comprise more than three groups or as few as two groups depending on the separation characteristics of the predictive algorithm. A person familiar with the art will be able to determine the optimal binning strategy depending on the distributions of class probability scores developed by modeling.

As used herein, the term "metastasis" refers to the recurrence or disease progression that may occur locally, regionally (such as nodal metastasis), or distally (such as distant metastasis to the brain, lung and other tissues). Class 1 or class 2 of metastasis, as used herein, includes low-risk (class 1; for example, having a 5-year relapse-free survival rate, a 5-year metastasis free survival rate, or a 5-year disease specific survival rate of greater than 50%, 55%, 60%, 65%, 70%, 75%, 80%, 85%, 90%, 95%, or more than 95%) or high-risk (class 2; for example, having a 5-year metastasis free survival rate, or a 5-year disease specific survival rate of less than 50%, 45%, 40%, 35%, 30%, 25%, 20%, 15%, 10%, 5%, or less than 5%) of metastasis according to any of the statistical methods disclosed herein. Class A, Class B, or Class C of metastasis, as used herein, includes low-risk (class A; for example having a recurrence risk of less than 25%, 20%, 15%, 10%, 5%, or less than 5%), intermediated risk (class B; for example having a recurrence risk of 75%, 70%, 65%, 60%, 55%, 50%, 45%, 40%, 35%, 30%, 25%, or any number in between) or high-risk (class C; for example, having a recurrence risk of 75%, 80%, 85%, 90%, 95%, or higher than 95%) of metastasis according to any of the statistical methods disclosed herein. The term "distant metastasis" as used herein, refers to metastases from a primary cSCC tumor that are disseminated widely. Patients with distant metastases require aggressive treatments, which can eradicate metastatic cSCC, prolong life, and/or cure some patients.

As used herein, the terms "locoregional recurrence" and "local recurrence" can be used interchangeably and refer to cancer cells that have spread to tissue immediately surrounding the primary cSCC tumor or were not completely ablated or removed by previous treatment or surgical resection. Locoregional recurrences are typically resistant to chemotherapy and radiation therapy. Locoregional recurrence can be difficult to control and/or treat if: (1) the primary cSCC tumor is located or involves a vital organ or structure that limits the potential for treatment; (2) recurrence after surgery or other therapy occurs, because while likely not a result from metastasis, high rates of recurrence indicate an advanced cSCC tumor; and (3) presence of lymph node metastases, while rare in cSCC, indicate advanced disease.

In some embodiments, the methods described herein can comprise determining that the cSCC tumor has an increased risk of metastasis or decreased overall survival by combining with clinical staging factors recommended by, for example, the American Joint Committee on Cancer (AJCC), the Brigham Women's Hospital (BWH), or the National Comprehensive Cancer Network (NCCN), to stage the primary cSCC tumor, or other histological features associated with risk of cSCC tumor metastasis or disease-related death.

As used herein, the terms "cutaneous squamous cell carcinoma" or "cSCC" or "SCC" refer to any cutaneous squamous cell carcinoma, regardless of tumor size, in patients without clinical or histologic evidence of regional or distant metastatic disease. A cutaneous squamous cell carcinoma sample may be obtained through a variety of sampling methods such as punch biopsy, shave biopsy, surgical excision, core needle biopsy, incisional biopsy, endoscope ultrasound (EUS) guided-fine needle aspirate (FNA) biopsy, percutaneous biopsy, and other means of extracting RNA from the primary cSCC tumor. A carcinoma is a type of cancer that develops from epithelial cells. Specifically, a carcinoma is a cancer that begins in a tissue that lines the inner or outer surfaces of the body, and that arises from cells originating in the endodermal, mesodermal, and ectodermal germ layer during embryogenesis. Squamous cell carcinomas have observable features and characteristics indicative of squamous differentiation (e.g., intercellular bridges, keratinization, squamous pearls). The most recognized risk factor for cSCC is exposure to sunlight; thus, most cSCC tumors develop on sun-exposed skin sites, for example, the head or neck area. They can also be found on the face, ears, lips, trunk, arms, legs, hands, or feet. Squamous cell carcinoma is the second most common skin cancer.

As used herein, "overall survival" (OS) refers to the percentage of people in a study or treatment group who are still alive for a certain period of time after they were diagnosed with or started treatment for a disease, such as cancer. The overall survival rate is often stated as a five-year survival rate, which is the percentage of people in a study or treatment group who are alive five years after their diagnosis or the start of treatment.

The phrase "measuring the gene-expression levels" or "determining the gene-expression levels," as used herein, refers to determining or quantifying RNA or proteins expressed by the gene or genes. The term "RNA" includes mRNA transcripts, and/or specific spliced variants of mRNA. The term "RNA product of the gene," as used herein, refers to RNA transcripts transcribed from the gene and/or specific spliced variants. In some embodiments, mRNA is converted to cDNA before the gene expression levels are measured. With respect to proteins, gene expression refers to proteins translated from the RNA transcripts transcribed from the gene. The term "protein product of the gene" refers to proteins translated from RNA products of the gene. A number of methods can be used to detect or quantify the level of RNA products of the gene or genes within a sample, including microarrays, Real-Time PCR (RT-PCR; including quantitative RT-PCR), nuclease protection assays, RNA-sequencing, and Northern blot analyses. In one embodiment, the assay uses the APPLIED BIOSYSTEMS^{™} HT7900 fast Real-Time PCR system. In addition, a person skilled in the art will appreciate that a number of methods can be used to determine the amount of a protein product of a gene of the methods disclosed herein, including immunoassays such as Western blots, ELISA, and immunoprecipitation followed by SDS-PAGE and immunocytochemistry. In certain embodiments, the expression level of each gene in the gene set is determined by reverse transcribing the isolated mRNA into cDNA and measuring a level of fluorescence for each gene in the gene set by a nucleic acid sequence detection system following Real-Time Polymerase Chain Reaction (RT-PCR).

A person skilled in the art will appreciate that a number of detection agents can be used to determine gene expression. For example, to detect RNA products of the biomarkers, probes, primers, complementary nucleotide sequences, or nucleotide sequences that hybridize to the RNA products can be used. In another example, to detect cDNA products of the biomarkers, probes, primers, complementary nucleotide sequences, or nucleotide sequences that hybridize to the cDNA products can be used. To detect protein products of the biomarkers, ligands or antibodies that specifically bind to the protein products can be used.

As used herein, the term "hybridize" refers to the sequence specific non-covalent binding interaction with a complementary nucleic acid. In one embodiment, the hybridization is under high stringency conditions. Appropriate stringency conditions that promote hybridization are known to those skilled in the art.

As used herein, the terms "probe" and "primer" refer to a nucleic acid sequence that will hybridize to a nucleic acid target sequence. In one example, the probe and/or primer hybridizes to an RNA product of the gene or a complementary nucleic acid sequence. In another example, the probe and/or primer hybridizes to a cDNA product. The length of probe or primer depends on the hybridizing conditions and the sequences of the probe or primer and nucleic acid target sequence. In one embodiment, the probe or primer is at least 8, 10, 15, 20, 25, 50, 75, 100, 150, 200, 250, 400, 500, or more than 500 nucleotides in length. Probes and/or primers may include one or more label. Probes and/or primers may be commercially sourced from various providers (e.g., ThermoFisher Scientific). In certain embodiments, a label may be any substance capable of aiding a machine, detector, sensor, device, or enhanced or unenhanced human eye from differentiating a labeled composition from an unlabeled composition. Examples of labels include, but are not limited to: a radioactive isotope or chelate thereof, dye (fluorescent or non-fluorescent), stain, enzyme, or nonradioactive metal. Specific examples include, but are not limited to: fluorescein, biotin, digoxigenin, alkaline phosphates, biotin, streptavidin, ³H, ¹⁴C, ³²P, ³⁵S, or any other compound capable of emitting radiation, rhodamine, 4-(4'-dimethylamino-phenylazo)benzoic acid; 4-(4'-dimethylamino-phenylazo)sulfonic acid (sulfonyl chloride); 5-((2-aminoethyl)-amino)-naphtalene-1-sulfonic acid; Psoralene derivatives, haptens, cyanines, acridines, fluorescent rhodol derivatives, cholesterol derivatives; ethylene-diamine-tetra-acetic acid and derivatives thereof, or any other compound that may be differentially detected. The label may also include one or more fluorescent dyes. Examples of dyes include, but are not limited to: CAL-Fluor Red 610, CAL-Fluor Orange 560, dR110, 5-FAM, 6FAM, dR6G, JOE, HEX, VIC, TET, dTAMRA, TAMRA, NED, dROX, PET, BHQ+, Gold540, and LIZ.

As used herein, a "sequence detection system" is any computational method in the art that can be used to analyze the results of a PCR reaction. One example is the APPLIED BIOSYSTEMS^{™} HT7900 fast Real-Time PCR system. In certain embodiments, gene expression can be analyzed using, e.g., direct DNA expression in microarray, Sanger sequencing analysis, Northern blot, the NANOSTRING^{®} technology, serial analysis of gene expression (SAGE), RNA-seq, tissue microarray, or protein expression with immunohistochemistry or western blot technique. PCR generally involves the mixing of a nucleic acid sample, two or more primers that are designed to recognize the template DNA, a DNA polymerase, which may be a thermostable DNA polymerase such as Taq or Pfu, and deoxyribose nucleoside triphosphates (dNTP's). Reverse transcription PCR, quantitative reverse transcription PCR, and quantitative real time reverse transcription PCR are other specific examples of PCR. In real-time PCR analysis, additional reagents, methods, optical detection systems, and devices known in the art are used that allow a measurement of the magnitude of fluorescence in proportion to concentration of amplified DNA. In such analyses, incorporation of fluorescent dye into the amplified strands may be detected or measured. In one embodiment, the expression level of each gene in the gene set is determined by reverse transcribing the isolated mRNA into cDNA and measuring a level of fluorescence for each gene in the gene set by a nucleic acid sequence detection system following Real-Time Polymerase Chain Reaction (RT-PCR).

As used herein, the terms "differentially expressed" or "differential expression" refer to a difference in the level of expression of the genes that can be assayed by measuring the level of expression of the products of the genes, such as the difference in level of messenger RNA transcript expressed (or converted cDNA) or proteins expressed of the genes. In one embodiment, the difference can be statistically significant. The term "difference in the level of expression" refers to an increase or decrease in the measurable expression level of a given gene as measured by the amount of messenger RNA transcript (or converted cDNA) and/or the amount of protein in a sample as compared with the measurable expression level of a given gene in a control, or control gene or genes in the same sample (for example, a non-recurrence sample). In another embodiment, the differential expression can be compared using the ratio of the level of expression of a given gene or genes as compared with the expression level of the given gene or genes of a control, wherein the ratio is not equal to 1.0. For example, an RNA, cDNA, or protein is differentially expressed if the ratio of the level of expression in a first sample as compared with a second sample is greater than or less than 1.0. For example, a ratio of greater than 1, 1.2, 1.5, 1.7, 2, 3, 3, 5, 10, 15, 20, or more than 20, or a ratio less than 1, 0.8, 0.6, 0.4, 0.2, 0.1, 0.05, 0.001, or less than 0.0001. In yet another embodiment, the differential expression is measured using p-value. For instance, when using p-value, a biomarker is identified as being differentially expressed as between a first sample and a second sample when the p-value is less than 0.1, less than 0.05, less than 0.01, less than 0.005, or less than 0.001.

The terms "increased expression" or "decreased expression," as used herein, refer to an expression level of one or more genes, or prognostic RNA transcripts, or their corresponding cDNAs, or their expression products that has been found to be differentially expressed in recurrent versus non-recurrent cSCC tumors. The higher the expression level of a gene that predominantly has increased expression in tumors of patients who had recurrence, the higher is the likelihood that the patient suffering from this tumor is expected to have a poor clinical outcome (i.e., higher risk of recurrence, metastasis, or both). In contrast, the lower the expression level of a gene that predominantly has increased expressed in tumors of patients who have recurrent tumors, the higher is the likelihood that the patient suffering from this tumor is expected to have a promising clinical outcome (i.e., decreased risk of recurrence, metastasis, or both). The lower the expression level of a gene that predominantly has decreased expression in tumors of patients who had recurrence, the higher is the likelihood that the patient suffering from this tumor is expected to have a poor clinical outcome (i.e., higher risk of recurrence, metastasis, or both). In contrast, the higher the expression level of a gene that predominantly has decreased expressed in tumors of patients who have recurrent tumors, the higher is the likelihood that the patient suffering from this tumor is expected to have a promising clinical outcome (i.e., decreased risk of recurrence, metastasis, or both).

References herein to the "same" level of biomarker indicate that the level of biomarker measured in each sample is identical (*i.e.,* when compared to the selected reference). References herein to a "similar" level of biomarker indicate that levels are not identical but the difference between them is not statistically significant (*i.e*., the levels have comparable quantities).

As used herein, the terms "control" and "standard" refer to a specific value that one can use to determine the value obtained from the sample. In one embodiment, a dataset may be obtained from samples from a group of subjects known to have a cutaneous squamous cell carcinoma or subtype. The expression data of the genes in the dataset can be used to create a control (standard) value that is used in testing samples from new subjects. In such an embodiment, the "control" or "standard" is a predetermined value for each gene or set of genes obtained from subjects with a cutaneous squamous cell carcinoma whose gene expression values and tumor types are known. In certain embodiments of the methods disclosed herein, non-limiting examples of control genes can include, but are not limited to, BAG6 (probe ID: Hs00190383), KMT2D/MLL2 (probe ID: Hs00912419_m1), MDM2 (probe ID: Hs00540450_s1), FXR1 (probe ID: Hs01096876_g1), KMT2C (probe ID: Hs01005521_m1), MDM4 (probe ID: Hs00967238_m1), VIM, and NF1B. In some embodiments, a control population may comprise healthy individuals, individuals with cancer, or a mixed population of individuals with or without cancer. In certain embodiments, a control population may comprise individuals with non-metastatic cancer or cancer that did not recur.

As used herein, the term "normal" when used with respect to a sample population refers to an individual or group of individuals that does/do not have a particular disease or condition (*e.g*., cSCC or recurrent cSCC) and is also not suspected of having or being at risk for developing the disease or condition. The term "normal" is also used herein to qualify a biological specimen or sample (*e.g*., a biological fluid) isolated from a normal or healthy individual or subject (or group of such subjects), for example, a "normal control sample." The "normal" level of expression of a marker is the level of expression of the marker in cells in a similar environment or response situation, in a patient not afflicted with cancer. A normal level of expression of a marker may also refer to the level of expression of a "reference sample" (*e.g*., a sample from a healthy subject not having the marker associated disease). A reference sample expression may be comprised of an expression level of one or more markers from a reference database. Alternatively, a "normal" level of expression of a marker is the level of expression of the marker in non-tumor cells in a similar environment or response situation from the same patient that the tumor is derived from.

As used herein, the terms "gene-expression profile," "GEP," or "gene-expression profile signature" refer to any combination of genes, the measured messenger RNA transcript expression levels, cDNA levels, or direct DNA expression levels, or immunohistochemistry levels of which can be used to distinguish between two biologically different corporal tissues and/or cells and/or cellular changes. In certain embodiments, a gene-expression profile is comprised of the gene-expression levels of at least 140, 139, 138, 137, 136, 135, 134, 133, 132,131, 130, 129, 128, 127, 126, 125, 124, 123, 122, 121, 120, 119, 118, 117, 116, 115, 114, 113, 112, 111, 110, 109, 108, 107, 106, 105, 104, 103, 102, 101, 100, 99, 98, 97, 96, 95, 94, 93, 92, 91, 90, 89, 88, 87, 86, 85, 84, 83, 82, 81, 80, 79, 78, 77, 76, 75, 74, 73, 72, 71, 70, 69, 68, 67, 66, 65, 64, 63, 62, 61, 60, 59, 58, 57, 56, 55, 54, 53, 52, 51, 50, 49, 48, 47, 46, 45, 44, 43, 42, 41, 40, 39, 38, 37, 36, 35, 34, 33, 32, 31, 30, 29, 28, 27, 26, 25, 24, 23, 22, 21, or 20genes. In one embodiment, the gene-expression profile is comprised of 56 genes. In another embodiment, the gene-expression profile is comprised of 40 genes. In another embodiment, the gene-expression profile is comprised of 30 genes. In another embodiment, the gene-expression profile is comprised of 20 genes. Additional genes for the gene set are optionally selected from the group consisting of: ACSBG1, AIM2, ANXA9, APOBEC3G, ARPC2, ATP6AP1, ATP6V0E2, BLOC1S1, C1QL4, C21orf59, C3orf70, CCL27, CD163, CEP76, CHI3L1, CHMP2B, CXCL10, CXCR4, CYP2D6 (LOC101929829), DARS, DCT, DDAH1, DSS1, DUXAP8, EGFR, EphB2, FCHSD1, FDFT1, FLG, FN1, HNRNPL, HOXA10 (HOXA9, MIR196B), HPGD, IL24, IL2RB, IL7R, INHBA, IPO5P1, KIT, KLK5, KRT17, KRT18, KRT19, KRT6B, LAMC2, LIME1 (ZGPAT), LOC101927502, LOR, LRRC47, MIER2, MIR129-1, MIR3916, MKLN1, MMP1, MMP12, MMP13, MMP3, MMP7, MMP9, MRC1, MRPL21, MYC, NEB, NEFL, NFIA, NFIB, NOA 1, PD1, PDL1, PI3, PIG3, PIGBOS1, PIM2, PLAU, PLS3, PTHLH, PTRHD1, RBM33, RNF135, RPL26L1, S100A8, S100A9, SEPT3, SERPINB2, SERPINB4, SLC1A3, SLC25A11, SNORD124, SPATA41, SPP1, THYN1, TMEM41B, TNNC1, TUBB3, TUFM (MIR4721), TYRP1, UGP2, USP7, VIM, YKT6, ZNF839, and/or ZSCAN31. In some embodiments, the gene set comprises 30 genes, or 40 genes. In some embodiments, the gene set further comprises control genes or normalization genes selected from: BAG6 (probe ID: Hs00190383), KMT2D/MLL2 (probe ID: Hs00912419_m1), MDM2 (probe ID: Hs00540450_s1), FXR1 (probe ID: Hs01096876_g1), KMT2C (probe ID: Hs01005521_m1), MDM4 (probe ID: Hs00967238_m1), VIM, and NF1B.

As used herein, the term "predictive training set" refers to a cohort of cSCC tumors with known clinical outcome for local recurrence, distant metastasis, or both and known genetic expression profile, used to define or establish all other cSCC tumors, based upon the genetic expression profile of each, as a low-risk, class 1 tumor type or a high-risk, class 2 tumor type. Additionally, included in the predictive training set is the definition of "threshold points," which are points at which a classification of metastatic risk is determined, specific to each individual gene expression level.

As used herein, the term "altered in a predictive manner" refers to changes in genetic expression profile that predict local recurrence, distant metastasis, metastatic risk, or predict overall survival. Predictive modeling risk assessment can be measured as: 1) a binary outcome having risk of metastasis or overall survival that is classified as low risk (*e.g*., termed Class 1 herein) vs. high risk (*e.g*., termed Class 2 herein); and/or 2) a linear outcome based upon a probability score from 0 to 1 that reflects the correlation of the genetic expression profile of a cSCC tumor with the genetic expression profile of the samples that comprise the training set used to predict risk outcome. Within the probability score range from 0 to 1, a probability score, for example, of less than 0.5 reflects a tumor sample with a low risk of local recurrence, metastasis, or death from disease, while a probability score, for example, of greater than 0.5 reflects a tumor sample with a high risk of local recurrence, metastasis, or death from disease. The increasing probability score from 0 to 1 reflects incrementally declining metastasis free survival. In one embodiment, the probability score is a bimodal, two-class analysis, wherein a patient having a value of between 0 and 0.499 is designated as class 1 (low risk; for example, having a 5-year relapse-free survival rate, a 5-year metastasis free survival rate, or a 5-year disease specific survival rate of greater than 50%, 55%, 60%, 65%, 70%, 75%, 80%, 85%, 90%, 95%, or more than 95%) and a patient having a value of between 0.500 and 1.00 is designated as class 2 (high risk; for example, having a 5-year metastasis free survival rate, or a 5-year disease specific survival rate of less than 50%, 45%, 40%, 35%, 30%, 25%, 20%, 15%, 10%, 5%, or less than 5%).

In certain embodiments, the probability score is a tri-modal, three-class analysis, wherein patients are designated as class A (low risk; for example having a recurrence risk of less than 25%, 20%, 15%, 10%, 5%, or less than 5%), class B (intermediate risk; for example having a recurrence risk of 75%, 70%, 65%, 60%, 55%, 50%, 45%, 40%, 35%, 30%, 25%, or any number in between), or class C (high risk; for example, having a recurrence risk of 75%, 80%, 85%, 90%, 95%, or higher than 95%). To develop a ternary, or three-class system of risk assessment, with Class A having a low risk of metastasis or death from disease, Class B having an intermediate risk, and Class C having a high risk, the median probability score value for all low risk or high risk tumor samples in the training set was determined, and one standard deviation from the median was established as a numerical boundary to define low or high risk. For example, low risk cSCC tumors within the ternary classification system can have a 5-year metastasis free survival of 100% (*e.g*., Class A; with a probability score of 0-0.337), compared to high risk (*e.g*., Class C; with a probability score of 0.673-1) cSCC tumors which can have a 20% 5-year metastasis free survival. Cases falling outside of one standard deviation from the median low or high risk probability scores have an intermediate risk, and intermediate risk (Class B; with a probability score of 0.338-0.672) cSCC tumors can have a 55% 5-year metastasis free survival rate.

The TNM (Tumor-Node-Metastasis) status system is the most widely used cancer staging system among clinicians and is maintained by the American Joint Committee on Cancer (AJCC) and the International Union for Cancer Control (UICC). Cancer staging systems codify the extent of cancer to provide clinicians and patients with the means to quantify prognosis for individual patients and to compare groups of patients in clinical trials and who receive standard care around the world.

Local recurrence rates for cSCC have been reported to be 1-10%, but can be as high as 47% in patients who have cSCCs with high-risk clinical features. While the overall rate of metastasis is ~5%, this rate increases up to ~45% in patients with high-risk clinical features or who have already experienced a recurrence. After regional or distant metastasis occurs, prognosis is usually poor, with 5-year survival rates ranging from 26-34% and 10-year survival rates of 16%. Although the overall percentages of patients who die from cSCC (~1%) are low, the absolute number of deaths are estimated to be equal to or greater than those attributed to melanoma, due to the large number of yearly cSCC diagnoses (400,000-700,000 patients), and account for the majority of NMSC-related deaths. In effect, local and regional recurrence from primary cSCC tumors remains a significant health burden.

Cutaneous squamous cell carcinoma stems from interfollicular epidermal keratinocytes and can arise from precancerous lesions, the most common of which are actinic keratoses. Once the malignant cells enter the dermis, the cSCC becomes invasive. Squamous cell carcinoma can present as smooth or hyperkeratinized lesions that are pink or skin-colored. They can exhibit ulceration and bleed when traumatized. Risk factors that contribute to the development of cSCC include exposures to ultraviolet radiation, ionizing radiation, and chemicals, as well as increased age and male gender. Immunosuppressed individuals, those with a history of non-Hodgkin lymphoma, including chronic lymphocytic leukemia, those with certain genetic skin conditions, and those who have had organ transplants are at a significantly increased risk for developing cSCC. In fact, the latter group has risk up to 100 times that of the normal population. Some drugs used to treat other types of skin cancer (*e.g*., basal cell carcinoma (BCC), melanoma), including hedgehog, BRAF, and MET inhibitors, can also increase the propensity for cSCC. Small, low-risk lesions can be treated with cryosurgery, curettage and electrodessication, or surgery, while larger, higher risk lesions are generally treated with surgical excision or Mohs surgery. Radiotherapy can be used in conjunction with surgery if margins are not cleared surgically or if there is perineural invasion. If regional recurrence occurs, the lymph nodes are the primary site of involvement, accounting for -80-85% of cSCC recurrences, while distant metastasis occurs in -15-20% of patients.

Because the risk for local recurrence in clinically or pathologically high risk squamous cell carcinoma lesions can approach 45%, and the risk of metastasis then increases upon local recurrence, there has been an increased interest in more accurately identifying such lesions. As such, the National Comprehensive Cancer Network (NCCN) and American Joint Committee on Cancer (AJCC) have recently proposed parameters to distinguish high risk lesions and follow-up measures for these lesions. These high-risk features include tumor size and location ("mask" areas of the face and/or ear and non-glabrous lip), increased thickness or Clark's level, immunosuppression, recurrent lesions, sites of chronic inflammation or previous radiation, poor differentiation, and perineural invasion. However, high-risk cSCC definitions from different groups are discordant, with the AJCC classifying a majority of lesions as low-risk and NCCN classifying a majority as high-risk. Such discrepancies, especially in the T2a and T2b groups, have led to the proposal of alternative staging criteria that can better elucidate high risk cSCC cases. In effect, there is a clinically unmet need for better markers to identify high-risk lesions, particularly molecular biomarkers that can be objectively evaluated. The validated prognostic gene expression profiles disclosed herein could inform clinical decision-making on, for example: (1) preoperative surgical staging, based on shave biopsy; (2) adjuvant radiation, nodal staging, adjuvant systemic therapy to reduce regional/distant metastasis; and (3) improving identification of patients with cSCC who can benefit from surgical, radiation and immunotherapy interventions.

Squamous cell carcinoma that is predicted to have an increased risk of recurrence, progression, or metastasis can be treated with an aggressive cancer treatment regimen. Advanced cSCC may be defined under two headings: (1) locoregional disease; and/or (2) distant metastases. Locoregional disease can be difficult to control and/or treat if: (1) the primary cSCC has invaded into neuronal or vascular structures; (2) there is presence of lymph node metastases, which indicate advanced disease; or (3) distant metastases have been detected.

In certain embodiments, the expression level of: ALOX12 is decreased, BBC is increased, BHLHB9 is decreased, GTPBP2 is decreased, HDDC3 is increased, ID2 is decreased, LCE2B is decreased, LOC100287896 is increased, MMP10 is decreased, MSANTD4 is decreased, NFASC is decreased, NFIC is decreased, PDPN is increased, RCHY1 is increased, RPP38 is decreased, RUNX3 is increased, TAF6L is increased, TFAP2B is decreased, ZNF48 is increased, and ZNF496 is increased, when comparing a recurrent tumor sample to a non-recurrent tumor sample.

Also disclosed but not forming part of the invention is a method for treating a patient with cutaneous squamous cell carcinoma (cSCC) tumor is disclosed herein, the method comprising: (a) obtaining a cSCC tumor sample from the patient and isolating mRNA from the sample; (b) determining the expression level of at least 10 genes in a gene set; wherein the at least ten genes in the gene set are selected from: ACSBG1, AIM2, ALOX12, ANXA9, APOBEC3G, ARPC2, ATP6AP1, ATP6V0E2, BBC, BHLHB9, BLOC1S1, C1QL4, C21orf59, C3orf70, CCL27, CD163, CEP76, CHI3L1, CHMP2B, CXCL10, CXCR4, CYP2D6 (LOC101929829), DARS, DCT, DDAH1, DSS1, DUXAP8, EGFR, EphB2, FCHSD1, FDFT1, FLG, FN1, GTPBP2, HDDC3, HNRNPL, HOXA10 (HOXA9, MIR196B), HPGD, ID2, IL24, IL2RB, IL7R, INHBA, IPO5P1, KIT, KLK5, KRT17, KRT 18, KRT 19, KRT6B, LAMC2, LCE2B, LIME1 (ZGPAT), LOC100287896, LOC101927502, LOR, LRRC47, MIER2, MIR129-1, MIR3916, MKLN1, MMP1, MMP10, MMP12, MMP13, MMP3, MMP7, MMP9, MRC1, MRPL21, MSANTD4, MYC, NEB, NEFL, NFASC, NFIA, NFIB, NFIC, NOA1, PD1, PDL1, PDPN, PI3, PIG3, PIGBOS1, PIM2, PLAU, PLS3, PTHLH, PTRHD1, RBM33, RCHY1, RNF135, RPL26L1, RPP38, RUNX3, S100A8, S100A9, SEPT3, SERPINB2, SERPINB4, SLC1A3, SLC25A11, SNORD124, SPATA41, SPP1, TAF6L, TFAP2B, THYN1, TMEM41B, TNNC1, TUBB3, TUFM (MIR4721), TYRP1, UGP2, USP7, VIM, YKT6, ZNF48, ZNF496, ZNF839, and/or ZSCAN31; (c) providing an indication as to whether the cSCC tumor has a low risk to a high risk of local recurrence, distant metastasis, or both, based on the expression level of at least 10 genes generated in step (b); and (d) administering to the patient an aggressive treatment when the determination is made in the affirmative that the patient has a cSCC tumor with a high risk of local recurrence, distant metastasis, or both.

As used herein, the terms "treatment," "treat," or "treating" refer to a method of reducing the effects of a disease or condition or symptom of the disease or condition. Thus, in the methods disclosed herein, treatment can refer to a 5%, 10%, 20%, 30%, 40%, 50%, 60%, 70%, 80%, 90%, or 100% reduction in the severity of an established disease or condition or symptom of the disease or condition. For example, a method of treating a disease is considered to be a treatment if there is a 5% reduction in one or more symptoms of the disease in a subject as compared to a control. Thus, the reduction can be a 5%, 10%, 20%, 30%, 40%, 50%, 60%, 70%, 80%, 90%, 100% or any percent reduction between 5% and 100% as compared to native or control levels. It is understood that treatment does not necessarily refer to a cure or complete ablation of the disease, condition, or symptoms of the disease or condition. After a cSCC is found and staged, a medical professional or team of medical professionals will recommend one or several treatment options. In determining a treatment plan, factors to consider include the type, location, and stage of the cancer, as well as the patient's overall physical health. Prior to the initiation of treatment and or therapy, all patients should be evaluated and managed by a multidisciplinary team with expertise and experience in cSCC. Patients with cSCC typically have a multidisciplinary health care team made up of doctors from different specialties, such as: a dermatologist (in particular, a dermatologist who specializes in Mohs micrographic surgery), an orthopedic surgeon (in particular, a surgeon who specializes in diseases of the bones, muscles, and joints), a surgical oncologist, a thoracic surgeon, a medical oncologist, a radiation oncologist, and/or a physiatrist (or rehabilitation doctor). After a cSCC is found and staged, a medical professional or team of medical professionals will typically recommend one or several treatment options including one or more of surgery, radiation, chemotherapy, and targeted therapy.

Low risk cSCC tumors as defined in the NCCN Guidelines^{®} involve: (1) an area of less than 20 mm (for truck and extremities) or less than 10 mm for the cheeks, forehead, scalp, neck and pretibial; (2) well defined borders; (3) primary cSCC tumor; (4) not rapidly growing; (5) from a patient who has no neurologic symptoms and is not considered immunosuppressed; (6) from a site free of chronic inflammation; (7) well or moderately differentiated; (8) free of acantholytic, adenosquamous, desmoplastic, or metaplastic subtypes; (9) depths of less than 2 mm; and (10) free of perineural, lymphatic, or vascular involvement.

High risk cSCC tumors as defined in the NCCN Guidelines^{®} involve: (1) an area of greater than 20 mm (for truck and extremities), greater than 10 mm for the cheeks, forehead, scalp, neck and pretibial, or any cSCC involving the "mask areas" (such as central face, eyelids, eyebrows, periorbital, nose, lips, chin, mandible, temple or ear), genitalia, hands and feet; (2) poorly defined borders; (3) recurrent cSCC tumor; (4) rapidly growing; (5) from a patient who has neurologic symptoms or is considered immunosuppressed; (6) from a site with chronic inflammation; (7) poorly differentiated; (8) presence of acantholytic, adenosquamous, desmoplastic, or metaplastic subtypes; (9) depths of greater than or equal 2 mm; and (10) presence of perineural, lymphatic, or vascular involvement.

As used herein, the term "aggressive cancer treatment regimen" refers to a treatment regimen that is determined by a medical professional or team of medical professionals and can be specific to each patient. A cSCC tumor predicted to have a high-risk of recurrence or a high-risk of metastasis, or a decreased chance of survival using the methods and kits disclosed herein, would be treated using an aggressive cancer treatment regimen. Whether a treatment is considered to be aggressive will generally depend on the cancer-type, the age of the patient, and other factors known to those of skill in the art. For example, in breast cancer, adjuvant chemotherapy is a common aggressive treatment given to complement the less aggressive standards of surgery and hormonal therapy. Those skilled in the art are familiar with various other aggressive and less aggressive treatments for each type of cancer. An aggressive cancer treatment regimen is defined by the National Comprehensive Cancer Network (NCCN), and has been defined in the NCCN Guidelines^{®} as including one or more of: 1) imaging (CT scan, PET/CT, MRI, chest X-ray), 2) discussion and/or offering of tumor resection if a tumor is determined to be resectable (e.g., by Mohs micrographic surgery or resection with complete circumferential margin assessment), 3) radiation therapy (RT), 4) chemoradiation, 5) chemotherapy, 6) regional limb therapy, 7) palliative surgery, 8) systemic therapy, 9) immunotherapy, and 10) inclusion in ongoing clinical trials. Guidelines for clinical practice are published in the National Comprehensive Cancer Network (NCCN Guidelines^{®} Squamous Cell Skin Cancer Version 2.2018, updated October 5, 2017, available on the World Wide Web at NCCN.org).

Additional therapeutic options may include, but are not limited to: 1) combination regimens such as: AD (doxorubicin, dacarbazine); AIM (doxorubicin, ifosfamide, mesna); MAID (mesna, doxorubicin, ifosfamide, dacarbazine); ifosfamide, epirubicin, mesna; gemcitabine and docetaxel; gemcitabine and vinorelbine; gemcitabine and dacarbazine; doxorubicin and olaratumab ; methotrexate and vinblastine; tamoxifen and sulindac; vincristine, dactinomycin, cylclophosphamide; vincristine, doxorubicin, cyclophosphamide; vincristine, doxorubicin, cyclophosphamide with ifosfamide and etoposide; vincristine, doxorubicin, ifosfamide; cyclophosphamide topotecan; or ifosfamide, doxorubicin; and/or 2) single agents, such as: cisplatin or other metallic compounds, 5-FU/capecitabine (Xeloda^{®}), cetuximab (Erbitux^{®}), pembrolizumab (MK-3475), panitumumab (Vectibix^{®}), dacomitinib (PF-00299804), gefitinib (ZD1839, Iressa), doxorubicin, ifosfamide, epirubicin, gemcitabine, dacarbazine, temozolomide, vinorelbine, eribulin, trabectedin, pazopanib, imatinib, sunitinib, regorafenib, sorafenib, nilotinib, dasatinib, interferon, toremifene, methotrexate, irinotecan, topotecan, paclitaxel, nab-paclitaxel (abraxane), docetaxel, bevacizumab, temozolomide, sirolimus (Rapamune^{®}), everolimus, temsirolimus, crizotinib, ceritinib, or palbociclib.

While surgical excision remains the mainstay for treating operable (Stage I-III) cSCC patients, for Stage I patients, en bloc resection with negative margins is generally considered sufficient for long-term local control. For those with incomplete excision margins and/or other unfavorable pathologic features, pre- or post-operative chemotherapy and/or radiation treatment can be recommended. No therapy has shown consistent efficacy for the treatment of excised cSCC, and treatment options for unresectable or advanced cSCC are limited.

Immunotherapy using an anti-PD1 inhibitor has shown promising results in early phase studies with cSCC patients. Examples of immunotherapies (that can be used alone or in combination with any one or more of tumor resection if a tumor is determined to be resectable, radiation therapy, chemoradiation, chemotherapy, regional limb therapy, palliative surgery, systemic therapy, additional immunotherapeutic, or inclusion in ongoing clinical trials), can include, for example, pembrolizumab (Keytruda^{®}) and nivolumab (Opdivo^{®}), cemiplimab (REGN2810; a fully human monoclonal antibody to Programmed Death-1). PD-1 is a protein on T-cells that normally help keep T-cells from attacking other cells in the body. By blocking PD-1, these drugs can boost the immune response against cancer cells. CTLA-4 inhibitors (for example, ipilimumab (Yervoy^{®})) are another class of drugs that can boost the immune response. In some instances, cytokine therapy (such as, interferon-alpha and interleukin-2) can be used to boost the immune system. Examples of interferon and interleukin-based treatments can include, but are not limited to, aldesleukin (proleukin^{®}), interferon alpha-2b (INTRON^{®}), and pegylated interferon alpha-2b (Sylvatron^{®}; PEG-INTRON^{®}, PEGASYS). For example, oncolytic virus therapy can be used. Along with killing the cells directly, the oncolytic viruses can also alert the immune system to attack the cancer cells. For example, talimogene laherparepvec (Imlygic^{®}), also known as T-VEC, is an oncolytic virus that can be used to treat melanomas. Additional immunotherapies may include CV8102.

Additionally, targeted therapies may be used to treat patients with cSCC. For example, targeted therapies can include, but are not limited to, vemurafenib (Zelboraf^{®}), dabrafenib (Tafinlar^{®}), trametinib (Mekinist^{®}), CLL442, and cobimetinib (Cotellic^{®}). These drugs target common genetic mutations, such as the BRAFV600 mutation, that may be found in a subset of cSCC patients.

In some embodiments, the cSCC tumor is a frozen sample. In another embodiment, the cSCC sample is formalin-fixed and paraffin embedded. In certain embodiments, the cSCC sample is taken from a formalin-fixed, paraffin embedded wide local excision sample. In another embodiment, the cSCC tumor is taken from a formalin-fixed, paraffin embedded primary biopsy sample. The cSCC sample has optionally been obtained from image guided surgical biopsy, shave biopsy, wide excision, or a lymph node dissection.

In certain embodiments, analysis of genetic expression and determination of outcome is carried out using radial basis machine and/or partial least squares analysis (PLS), partition tree analysis, logistic regression analysis (LRA), K-nearest neighbor, neural networks, ensemble learners, voting algorithms, or other algorithmic approach. These analysis techniques take into account the large number of samples required to generate a training set that will enable accurate prediction of outcomes as a result of cut-points established with an in-process training set or cut-points defined for non-algorithmic analysis, but that any number of linear and nonlinear approaches can produce a statistically significant and clinically significant result. As used herein, the term "Kaplan-Meier survival analysis" is understood in the art to be also known as the product limit estimator, which is used to estimate the survival function from lifetime data. In medical research, it is often used to measure the fraction of patients living for a certain amount of time after treatment. JMP GENOMICS^{®}, R, Python libraries including SciPy, SciKit, and numpy software or systems such as TensorFlow provides an interface for utilizing each of the predictive modeling methods disclosed herein, and should not limit the claims to methods performed only with JMP GENOMICS^{®}, R, Python, or TensorFlow software.

Also disclosed but not forming part of the present invention is a kit comprising primer pairs suitable for the detection and quantification of nucleic acid expression of at least ten genes is disclosed herein, wherein the at least ten genes are selected from: ACSBG1, AIM2, ALOX12, ANXA9, APOBEC3G, ARPC2, ATP6AP1, ATP6V0E2, BBC, BHLHB9, BLOC1S1, C1QL4, C21orf59, C3orf70, CCL27, CD163, CEP76, CHI3L1, CHMP2B, CXCL10, CXCR4, CYP2D6 (LOC101929829), DARS, DCT, DDAH1, DSS1, DUXAP8, EGFR, EphB2, FCHSD1, FDFT1, FLG, FN1, GTPBP2, HDDC3, HNRNPL, HOXA10 (HOXA9, MIR196B), HPGD, ID2, IL24, IL2RB, IL7R, INHBA, IPO5P1, KIT, KLK5, KRT17, KRT18, KRT19, KRT6B, LAMC2, LCE2B, LIME1 (ZGPAT), LOC100287896, LOC101927502, LOR, LRRC47, MIER2, MIR129-1, MIR3916, MKLN1, MMP1, MMP10, MMP 12, MMP13, MMP3, MMP7, MMP9, MRC1, MRPL21, MSANTD4, MYC, NEB, NEFL, NFASC, NFIA, NFIB, NFIC, NOA1, PD1, PDL1, PDPN, PI3, PIG3, PIGBOS1, PIM2, PLAU, PLS3, PTHLH, PTRHD1, RBM33, RCHY1, RNF135, RPL26L1, RPP38, RUNX3, S100A8, S100A9, SEPT3, SERPINB2, SERPINB4, SLC1A3, SLC25A11, SNORD124, SPATA41, SPP1, TAF6L, TFAP2B, THYN1, TMEM41B, TNNC1, TUBB3, TUFM (MIR4721), TYRP1, UGP2, USP7, VIM, YKT6, ZNF48, ZNF496, ZNF839, and/or ZSCAN31.

The primer pairs suitable for the detection and quantification of nucleic acid expression of at least ten genes are primer pairs for: ACSBG1, AIM2, ALOX12, ANXA9, APOBEC3G, ARPC2, ATP6AP1, ATP6V0E2, BBC, BHLHB9, BLOC1S1, C1QL4, C21orf59, C3orf70, CCL27, CD163, CEP76, CHI3L1, CHMP2B, CXCL10, CXCR4, CYP2D6 (LOC101929829), DARS, DCT, DDAH1, DSS1, DUXAP8, EGFR, EphB2, FCHSD1, FDFT1, FLG, FN1, GTPBP2, HDDC3, HNRNPL, HOXA10 (HOXA9, MIR196B), HPGD, ID2, IL24, IL2RB, IL7R, INHBA, IPO5P1, KIT, KLK5, KRT17, KRT 18, KRT 19, KRT6B, LAMC2, LCE2B, LIME1 (ZGPAT), LOC100287896, LOC101927502, LOR, LRRC47, MIER2, MIR129-1, MIR3916, MKLN1, MMP1, MMP10, MMP 12, MMP13, MMP3, MMP7, MMP9, MRC1, MRPL21, MSANTD4, MYC, NEB, NEFL, NFASC, NFIA, NFIB, NFIC, NOA1, PD1, PDL1, PDPN, PI3, PIG3, PIGBOS1, PIM2, PLAU, PLS3, PTHLH, PTRHD1, RBM33, RCHY1, RNF135, RPL26L1, RPP38, RUNX3, S100A8, S100A9, SEPT3, SERPINB2, SERPINB4, SLC1A3, SLC25A11, SNORD124, SPATA41, SPP1, TAF6L, TFAP2B, THYN1, TMEM41B, TNNC1, TUBB3, TUFM (MIR4721), TYRP1, UGP2, USP7, VIM, YKT6, ZNF48, ZNF496, ZNF839, and/or ZSCAN31. The primer pairs optionally comprise primer pairs for 10 genes, 20 genes, 30 genes, 40 genes or more.

Also disclosed but not forming part of the invention are kits to be used in assessing the expression of a gene or set of genes in a cSCC sample or biological sample from a subject to assess the risk of developing recurrence, metastasis, or both. Also disclosed but not forming part of the invention is a kit comprising primer pairs suitable for the detection and quantification of nucleic acid expression of at least ten genes selected from: ACSBG1, AIM2, ALOX12, ANXA9, APOBEC3G, ARPC2, ATP6AP1, ATP6V0E2, BBC, BHLHB9, BLOC1S1, C1QL4, C21orf59, C3orf70, CCL27, CD163, CEP76, CHI3L1, CHMP2B, CXCL10, CXCR4, CYP2D6 (LOC101929829), DARS, DCT, DDAH1, DSS1, DUXAP8, EGFR, EphB2, FCHSD1, FDFT1, FLG, FN1, GTPBP2, HDDC3, HNRNPL, HOXA10 (HOXA9, MIR196B), HPGD, ID2, IL24, IL2RB, IL7R, INHBA, IPO5P1, KIT, KLK5, KRT17, KRT18, KRT19, KRT6B, LAMC2, LCE2B, LIME1 (ZGPAT), LOC100287896, LOC101927502, LOR, LRRC47, MIER2, MIR129-1, MIR3916, MKLN1, MMP1, MMP10, MMP 12, MMP13, MMP3, MMP7, MMP9, MRC1, MRPL21, MSANTD4, MYC, NEB, NEFL, NFASC, NFIA, NFIB, NFIC, NOA1, PD1, PDL1, PDPN, PI3, PIG3, PIGBOS1, PIM2, PLAU, PLS3, PTHLH, PTRHD1, RBM33, RCHY1, RNF135, RPL26L1, RPP38, RUNX3, S100A8, S100A9, SEPT3, SERPINB2, SERPINB4, SLC1A3, SLC25A11, SNORD124, SPATA41, SPP1, TAF6L, TFAP2B, THYN1, TMEM41B, TNNC1, TUBB3, TUFM (MIR4721), TYRP1, UGP2, USP7, VIM, YKT6, ZNF48, ZNF496, ZNF839, and/or ZSCAN31.

Kits can include any combination of components that facilitates the performance of an assay. A kit that facilitates assessing the expression of the gene or genes may include suitable nucleic acid-based and/or immunological reagents as well as suitable buffers, control reagents, and printed protocols. A "kit" is any article of manufacture (*e.g*., a package or container) comprising at least one reagent, *e.g.,* a probe or primer set, for specifically detecting a marker or set of markers used in the methods disclosed herein. The article of manufacture may be promoted, distributed, sold, or offered for sale as a unit for performing the methods disclosed herein. The reagents included in such a kit comprise probes, primers, or antibodies for use in detecting one or more of the genes and/or gene sets disclosed herein and demonstrated to be useful for predicting recurrence, metastasis, or both, in patients with cSCC. Kits that facilitate nucleic acid based methods may further include one or more of the following: specific nucleic acids such as oligonucleotides, labeling reagents, enzymes including PCR amplification reagents such as Taq or Pfu, reverse transcriptase, or other, and/or reagents that facilitate hybridization. In addition, the kits disclosed herein may preferably contain instructions which describe a suitable detection assay. Such kits can be conveniently used, *e.g*., in clinical settings, to diagnose and evaluate patients exhibiting symptoms of cancer, in particular patients exhibiting the possible presence of a cutaneous squamous cell carcinoma.

### EXAMPLES

The Examples that follow are illustrative of specific embodiments of the claimed invention, and various uses thereof. They are set forth for explanatory purposes only, and should not be construed as limiting the scope of the claimed invention in any way.

### Example 1: cSSC tumor sample preparation and expression analysis

### a. cSCC tumor sample preparation and RNA isolation

Formalin-fixed paraffin embedded (FFPE) primary squamous cell carcinoma tumor specimens arranged in 5 µm sections on microscope slides were acquired from multiple institutions under Institutional Review Board (IRB) approved protocols. All tissue was reviewed by a pathologist. Tissue was marked and tumor tissue was dissected from the slide using a sterile disposable scalpel, collected into a microcentrifuge tube, and deparaffinized using xylene. RNA was isolated from each specimen using the QIAGEN QIAsymphony RNA kit (Hilden, Germany) on the QIAGEN QIAsymphony SP sample preparation automated extractor. RNA quantity was assessed using the NanoDrop^{™} 8000 system.

### b. cDNA generation and RT-PCR analysis

RNA isolated from FFPE samples was converted to cDNA using the Applied Biosystems High Capacity cDNA Reverse Transcription Kit (Life Technologies Corporation, Grand Island, NY). Prior to performing the RT-PCR assay, each cDNA sample underwent a 14-cycle pre-amplification step. Pre-amplified cDNA samples were diluted 20-fold in TE buffer. 7.5 µL of each diluted sample was mixed with 7.5 µL of TaqMan OpenArray Real-Time Mastermix, and the solution was loaded to a custom high throughput microfluidics OpenArray card containing primers specific for the genes. Each sample was run in triplicate. The gene expression profile test was performed on a ThermoFisher QuantStudio12k Flex Real-Time PCR system (Life Technologies Corporation, Grand Island, NY).

### c. Expression analysis and class assignment

Mean Ct values were calculated for triplicate sample sets, and ΔCₜ values were calculated by subtracting the mean Ct of each discriminating gene from the geometric mean of the mean Ct values of all endogenous control genes. ΔCₜ values were standardized according to the mean of the expression of all discriminant genes with a scale equivalent to the standard deviation. Various predictive modeling methods, including radial basis machine, k-nearest neighbor, partition tree, logistic regression, discriminant analysis and distance scoring, and neural network analysis were performed using R version 3.3.2.

### Example 2: cSCC metastatic risk genetic signature and biomarker expression

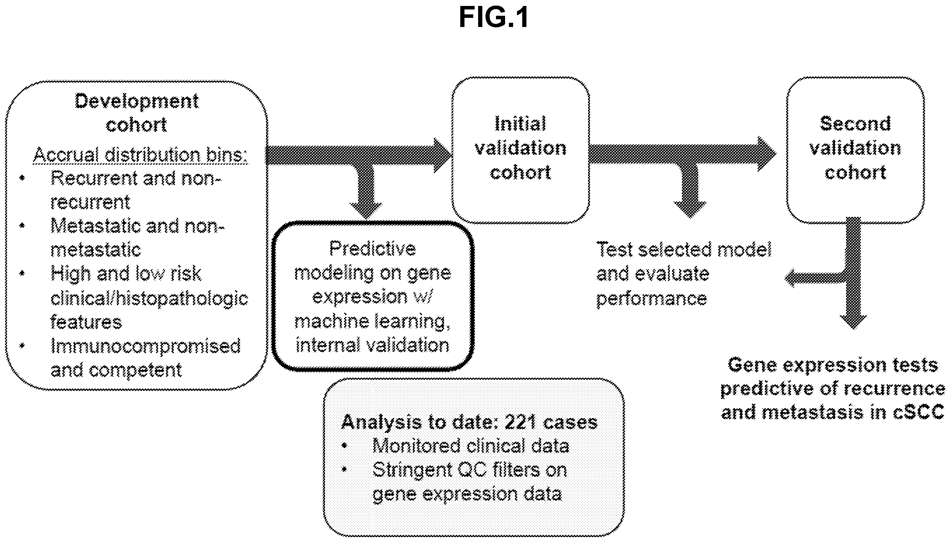

The study design workflow is shown in Figure 1. First, in order to develop the gene expression profile for cSCC prognostication, cases with annotated clinical data and sufficient follow-up were used as a development set. Pre-specified bins of patients within the recurrent and non-recurrent group were created, including immunocompromised, immunocompetent, those with a certain number of high risk features and low risk cases. The goal was to satisfy the pre-specified number of cases in each bin for development. Predictive modeling was performed on gene expression data from the development cohort. The predictive model was then validated. 221 cases were included in the development set *(see* Table 1). Table 1 shows the demographics for the cohort of 221 cases used in this study. They are also stratified by non-recurrence or recurrence. Recurrence is defined as any recurrence - local nodal (satellitosis through regional nodes and distant metastasis). Note that cases with R1 or R2 and local recurrence in the scar or contiguous to the scar were embargoed from this analysis. Characteristics that are associated with higher risk tumors (such as male sex, compromised immune system, head and neck primary tumor, poor differentiation or undifferentiated, higher Clark Level, perineural invasion, and invasion into subcutaneous fat) are features included. This is after embargoing cases that have not yet had data monitoring and did not meet very stringent gene expression data requirements.

**Table 1: Demographics for the cohort of 221 cases used in Examples 2 and 3**

| **Feature** | | **All (n=221)** | **Non-Recurrence (n=196)** | **With Recurrence (n=25)** | **p-value** |
|---|---|---|---|---|---|
| Age: Median years (range) | | 74 (43-97) | 74 (45-97) | 69 (43-91) | n.s. |
| Mean +/- SD | | 72.8 +/- 11.2 | 73.3 +/- 10.8 | 68.6 +/- 13.2 | |
| Definitive surgery: Mohs | | 181 (82%) | 161 (82%) | 20 (80%) | n.s. |
| | WLE | 39 (18%) | 34 (17%) | 5 (20%) | |
| Male sex | | 164 (74%) | 143 (73%) | 21 (84%) | n.s. |
| Patient immunocompromised | | 30 (14%) | 20 (10%) | 10 (40%) | p<0.001 |
| Located on head or neck | | 146 (66%) | 129 (66%) | 17 (68%) | n.s. |
| Tumor diameter: | | | | | p<0.001 |
| | Median cm (range) | 1.4 (0-28) | 1.15 (0-8.8) | 2.9 (0.25-28) | |
| | Mean +/- SD | 1.88 +/- 2.34 | 1.62 +/- 1.47 | 3.89 +/- 5.27 | |
| Differentiation Status: | | 12 (5%) | 9 (5%) | 3 (12%) | p<0.001 |
| | Poor / Undifferentiated | | | | |
| | Clark Level IV / V | 73 (33%) | 67 (34%) | 6 (24%) | p<0.001 |
| Perineural invasion present | | 12 (5%) | 9 (5%) | 3 (12%) | p<0.001 |
| Invasion into subcutaneous fat | | 22 (10%) | 19 (10%) | 3 (12%) | P=0.015 |

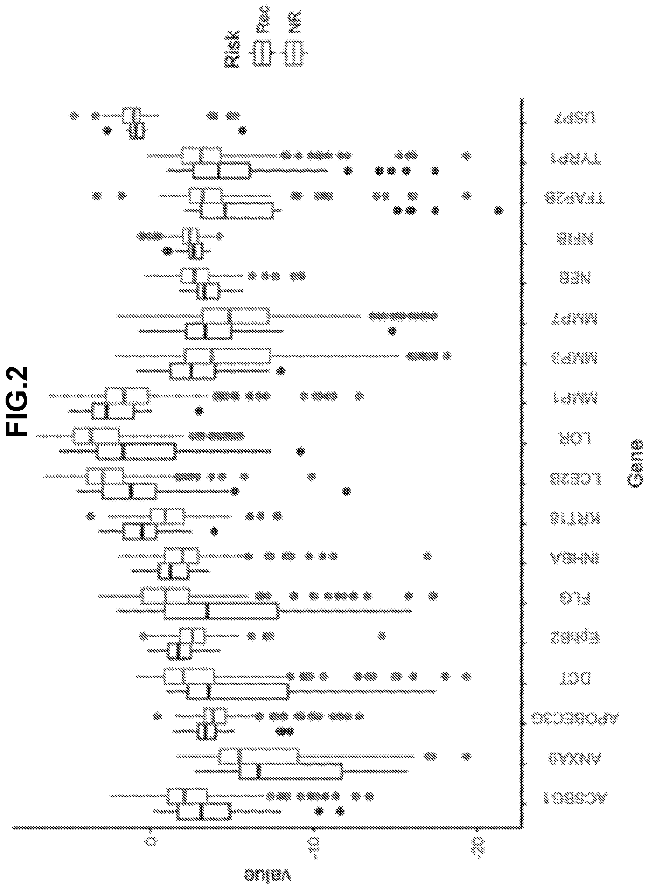

Gene expression differences (RT-PCR data from 73 genes) between recurrent and non-recurrent cSCC cases were evaluated. Using the gene expression data, several control genes were identified that had stable expression across all of the samples. These control genes were then used to normalize the expression of the remaining genes. Gene expression differences between recurrent and non-recurrent cases were investigated to find the genes that are significant. Significant gene expression differences that were associated with local recurrences, regional metastases, and distant metastases were also evaluated. Table 2 below shows genes associated with regional/distant metastases. Genetic expression of the discriminant genes in the signature (Table 2) was assessed in a cohort of 240 cSCC samples using RT-PCR, 18 of these were independently significant to a p-value of p<0.05 (*see* Figure 2). As shown in Table 3 below, of the 63 discriminating genes, 18 were altered in metastatic cSCC tumors compared to nonmetastatic tumors with a p-value of p<0.05.

**Table 2: 63 candidate genes for the GEP signature to predict metastatic risk and/recurrence in cSCC tumors**

| **Gene symbol** | ***p-value*** | **mean** - **Recurrence** | **mean** - **Non-Recurrence** |
|---|---|---|---|
| LOR | 0.000 | 0.310 | 2.935 |
| KRT18 | 0.000 | 0.454 | -1.081 |
| LCE2B | 0.000 | 0.447 | 2.490 |
| EphB2 | 0.001 | -1.846 | -2.648 |
| FLG | 0.001 | -4.104 | -1.594 |
| DCT | 0.001 | -5.696 | -3.003 |
| TFAP2B | 0.001 | -6.571 | -3.836 |
| NEB | 0.002 | -3.495 | -2.807 |
| TYRP1 | 0.006 | -5.869 | -3.709 |
| MMP3 | 0.006 | -2.887 | -5.159 |
| MMP7 | 0.010 | -3.915 | -5.568 |
| MMP1 | 0.014 | 2.290 | 0.970 |
| INHBA | 0.016 | -1.203 | -2.222 |
| ACSBG1 | 0.024 | -3.613 | -2.489 |
| USP7 | 0.029 | 0.689 | 1.076 |
| APOBEC3G | 0.035 | -3.800 | -4.260 |
| NFIB | 0.036 | -2.623 | -2.385 |
| ANXA9 | 0.050 | -8.007 | -7.059 |
| RCHY1 | 0.055 | -2.418 | -2.665 |
| PDPN | 0.056 | 1.672 | 1.251 |
| ALOX12 | 0.066 | -4.112 | -3.688 |
| YKT6 | 0.070 | 1.449 | 1.140 |
| PLAU | 0.091 | 1.873 | 1.913 |
| ID2 | 0.110 | -0.921 | -0.550 |
| MMP10 | 0.119 | -0.067 | -1.164 |
| HPGD | 0.141 | -6.830 | -5.781 |
| FN1 | 0.147 | 1.594 | 1.186 |
| HNRNPL | 0.156 | -0.036 | 0.085 |
| AIM2 | 0.159 | -4.093 | -4.610 |
| MMP13 | 0.178 | -7.096 | -8.365 |
| BBC | 0.179 | -7.464 | -7.738 |
| EGFR | 0.189 | 0.908 | 0.705 |
| SPP1 | 0.200 | -0.144 | -1.332 |
| SERPINB4 | 0.251 | -11.815 | -10.852 |
| NEFL | 0.292 | -2.876 | -1.321 |
| NFASC | 0.301 | -3.832 | -3.738 |
| PI3 | 0.324 | 4.686 | 4.847 |
| PIG3 | 0.333 | -3.420 | -3.698 |
| LAMC2 | 0.350 | 0.480 | 0.196 |
| ARPC2 | 0.353 | -0.053 | -0.006 |
| AADAC | 0.379 | -16.094 | -15.629 |
| IL24 | 0.387 | -3.969 | -4.629 |
| S100A8 | 0.388 | 3.838 | 3.936 |
| CCL27 | 0.398 | -12.922 | -13.230 |
| PTHLH | 0.401 | 0.777 | 0.907 |
| S100A9 | 0.457 | 7.525 | 7.534 |
| DDAH1 | 0.461 | -5.660 | -5.216 |
| PDL1 | 0.471 | -3.067 | -3.124 |
| DSS1 | 0.477 | -5.722 | -5.442 |
| KRT19 | 0.486 | -2.982 | -3.853 |
| KIT | 0.529 | -2.391 | -2.415 |
| TUBB3 | 0.599 | -4.541 | -5.167 |
| MYC | 0.631 | -3.816 | -3.749 |
| CHI3L1 | 0.653 | -0.029 | 0.025 |
| MMP9 | 0.684 | 1.649 | 1.733 |
| CXCR4 | 0.742 | -8.733 | -8.858 |
| ATP6V0E2 | 0.750 | -8.562 | -8.519 |
| CXCL10 | 0.785 | -3.039 | -3.184 |
| PD1 | 0.825 | -1.878 | -1.870 |
| IL7R | 0.872 | -8.524 | -8.094 |
| MMP12 | 0.919 | -2.958 | -3.225 |
| CEP76 | 0.981 | -4.361 | -4.656 |

**Table 3: 18 Genes included in a GEP signature able to predict recurrence in cSCC**

| **Gene symbol** | ***p-value*** | **mean** - **Recurrence** | **mean** - **Non-Recurrence** |
|---|---|---|---|
| ACSBG1 | 0.024 | -3.613 | -2.489 |
| ANXA9 | 0.050 | -8.007 | -7.059 |
| APOBEC3G | 0.035 | -3.800 | -4.260 |
| DCT | 0.001 | -5.696 | -3.003 |
| EphB2 | 0.001 | -1.846 | -2.648 |
| FLG | 0.001 | -4.104 | -1.594 |
| INHBA | 0.016 | -1.203 | -2.222 |
| KRT18 | 0.000 | 0.454 | -1.081 |
| LCE2B | 0.000 | 0.447 | 2.490 |
| LOR | 0.000 | 0.310 | 2.935 |
| MMP1 | 0.014 | 2.290 | 0.970 |
| MMP3 | 0.006 | -2.887 | -5.159 |
| MMP7 | 0.010 | -3.915 | -5.568 |
| NEB | 0.002 | -3.495 | -2.807 |
| NFIB | 0.036 | -2.623 | -2.385 |
| TFAP2B | 0.001 | -6.571 | -3.836 |
| TYRP1 | 0.006 | -5.869 | -3.709 |
| USP7 | 0.029 | 0.689 | 1.076 |

### Example 3: Initial training set development studies and comparison to validation cohort

R version 3.3.2 was used to train multiple predictive models (e.g., multiple machinelearning methods such as, neural networks, gradient boosting machine, generalized linear model boost, radial basis function, rule-based classification, decision tree classification, and/or regularized linear discriminant analysis) against the normalized Ct values obtained from RT-PCR analysis in 181 cSCC cases selected at random from the 240 cases in the combined set. The average of the top predictive models was more sensitive than either the Brigham and Women's Hospital (BWH) or American Joint Committee on Cancer (AJCC) models with minimal loss of specificity. These results show that recurrent and non-recurrent cSCC can be identified through gene expression profiling and gene expression can be used to identify cSCC patients with a higher risk of recurrence. A validated prognostic test could inform clinical decision-making on preoperative surgical staging (for example, based on shave biopsy), surgical approach (SLNB) or adjuvant radiation to reduce local recurrence, and adjuvant radiation, nodal staging, adjuvant systemic therapy to reduce regional/distant metastasis. Such a test could improve such intervention decisions and help determine which patients may benefit from additional therapeutic modalities.

**Table 4: Predictive modeling - local recurrence**

| **Local Recurrence** | **GEP Example 2** | **BWH** | **AJCCv7** | **AJCCv8** |
|---|---|---|---|---|
| **Sensitivity** | 75% | 17% | 0% | 39% |
| **Specificity** | 92% | 90% | 99.5% | 79% |
| **negative predictive value (NPV)** | 98% | 92% | 92% | 94% |
| **positive predictive value (PPV)** | 50% | 13% | 94% | 14% |

**Table 5: Predictive modeling - metastasis**

| **Regional/Distant Metastasis** | **GEP Example 2** | **BWH** | **AJCCv7** | **AJCCv8** |
|---|---|---|---|---|
| **Sensitivity** | 83% | 23% | 0% | 46% |
| **Specificity** | 95% | 90% | 100% | 79% |
| **negative predictive value (NPV)** | 99% | 95% | 94% | 96% |
| **positive predictive value (PPV)** | 53% | 13% | 0% | 12% |

### Example 4: Prognostic gene expression profile test in cSCC in patients with one or more high-risk features

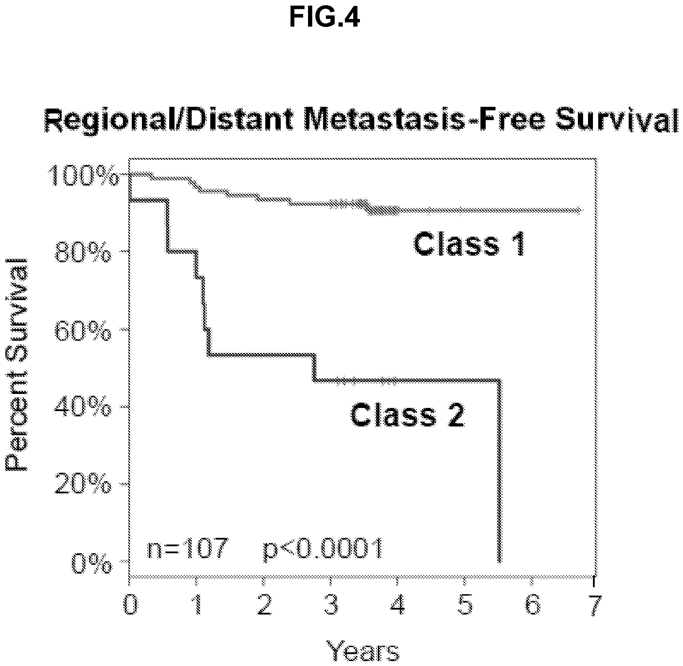

To identify a gene expression profile that accurately predicts: (1) primary cSCC with a high risk of regional nodal/distant metastasis; and (2) primary cSCC with high risk of local recurrence after complete surgical clearance, a multi-center study was performed using archived primary tissue samples with extensive capture of associated clinical data. The approach uses targeted candidate genes from the literature combined with genes from a global approach microarray screen. Samples are from subjects with pathologically confirmed cSCC diagnosed after 2006, minimum 3 years of follow-up or event (*see* Tables 6 and 7). Two separate outcomes were measured: (1) nodal/distant metastasis; and (2) local recurrence. Accuracy metrics demonstrate that the gene expression signature has prognostic value for in an independent cohort (*see* Table 8 and Figure 4). The prognostic test could inform clinical decision-making on: (1) preoperative surgical staging, based on shave biopsy; and (2) adjuvant radiation, nodal staging, adjuvant systemic therapy to reduce regional/distant metastasis.

**Table 6: Demographics for development stage of Example 4.**

| **Feature** | | **All (n=122)** | **Non-Metastatic (n=108)** | **Regional/distant metastasis (n=14)** |
|---|---|---|---|---|
| Age: Median years (range) | | 74 (49-97) | 74 (50-97) | 74.5 (49-91) |
| Definitive surgery: Mohs | | # 99 (82%) | # 88 (82%) | 11 (79%) |
| Male sex | | 94 (77%) | 81 (75%) | 13 (93%) |
| Patient immunocompromised | | 17 (14%) | 13 (12%) | 4 (29%) |
| Located on head or neck | | 87 (71%) | 77 (71%) | 10(71%) |
| Tumor diameter: Mean +/- SD | | 2.0 +/- 2.9 | 1.5 +/- 1.3 | 5.8 +/- 6.7*** |

| Differentiation Status: | | | | |
|---|---|---|---|---|
| | Poorly differentiated | 5 (4%) | 4 (4%) | 1 (7%) |
| | Clark Level IV / V | 45 (37%) | 40 (37%) | 5 (36%) |
| Perineural invasion present | | 7 (6%) | 6 (6%) | 1 (7%) |
| Invasion into subcutaneous fat | | 7 (6%) | 4 (4%) | 3 (21%) ** |
| ^{#}1 case with unknown surgery type; Wilcoxon F or Chi-square test p **<0.01 ***<0.001 | | | | |

**Table 7: Demographics for validation stage of Example 4.**

| **Feature** | | **All (n=107)** | **Non-Met (n=90)** | **Regional/distant met (n=17)** |
|---|---|---|---|---|
| Age: Median years (range) | | 72 (30-93) | 72.5 (45-93) | 72 (30-88) |
| Definitive surgery: Mohs | | # 86 (81%) | 76 (84%) | 10 (63%)^{#∗} |
| Male sex | | 78 (73%) | 64 (71%) | 14 (82%) |
| Patient immunocompromised | | 12 (11%) | 10 (11%) | 2 (12%) |
| Located on head or neck | | 76 (71%) | 62 (69%) | 14 (82%) |
| Tumor diameter: Mean +/- SD | | 1.9 +/- 1.7 | 1.9 +/- 1.2 | 3.3 +/- 2.6** |
| Differentiation Status: | | | | |
| | Poorly differentiated | 13 (12%) | 6 (7%) | 7 (42%)*** |
| | Clark Level IV / V | 32 (30%) | 25 (28%) | 7(41%) |
| Perineural invasion present | | 9 (8%) | 3 (3%) | 6 (35%)*** |
| Invasion into subcutaneous fat | | 17 (16%) | 11 (12%) | 6 (35%)^{∗} |
| ^{#}1 case with unknown surgery type; Wilcoxon F or Chi-square test p ^{∗}<0.05 ^{∗∗}<0.01 ^{∗∗∗}<0.001 | | | | |

**Table 8: Predictive modeling**

| **Metric** | **GEP Example 4** | **AJCC 8** | **BWH** |
|---|---|---|---|
| **Sensitivity** | 53% | 53% | 41% |
| **Specificity** | 93% | 87% | 88% |
| **negative predictive value (NPV)** | 91% | 91% | 89% |
| **positive predictive value (PPV)** | 60% | 43% | 39% |

### Example 5: Prognostic gene expression signature for risk assessment in cSCC with a subanalysis in the head and neck region

To identify a gene expression profile that accurately predicts: (1) primary cSCC with a high risk of regional nodal/distant metastasis; and (2) primary cSCC with high risk of local recurrence after complete surgical clearance, a multi-center study was performed using archived primary tissue samples with extensive capture of associated clinical data. The approach uses targeted candidate genes from the literature combined with genes from a global approach microarray screen. Samples are from subjects with pathologically confirmed cSCC diagnosed after 2006, minimum 3 years of follow-up or event (*see* Table 9). Two separate outcomes were measured: (1) nodal/distant metastasis; and (2) local recurrence. Accuracy metrics accuracy metrics for all and head and neck cSCC cases suggest that gene expression signature has prognostic value in an independent cohort (*see* Table 10). The prognostic signature with a robust PPV for high-risk disease will improve identification of patients with cSCC who can benefit from surgical, radiation and immunotherapy interventions.

**Table 9: Demographics - head and neck subanalysis**

| **Feature of head and neck case** | | **Non-Metastatic (n=34)** | **Metastasis (n=9)** |
|---|---|---|---|
| Age: Median years (range) | | 75 (49-89) | 77 (49-89) |
| Definitive surgery: Mohs | | 33 (97%) | 8 (89%) |
| Male sex | | 31 (91%) | 8 (89%) |
| Tumor diameter: Mean cm +/- SD | | 2.52 +/- 1.35 | 5.89 +/- 8.36 |
| Differentiation Status: | | | |
| | Poor / Undifferentiated | 2 (6%) | 2 (22%) |
| | Clark Level IV / V | 4 (12%) | 1 (11%) |
| Perineural invasion present | | 4 (12%) | 0 (0%) |
| Invasion into subcutaneous fat | | 7 (21%) | 1 (11%) |

**Table 10: Predictive modeling - head and neck subanalysis**

| | **All (n = 107)** | | **H&N (n = 76)** | |
|---|---|---|---|---|
| **Metric** | **GEP Example 5** | **BWH** | **GEP this study** | **BWH** |
| **Sensitivity** | 53% | 41% | 43% | 43% |
| **Specificity** | 93% | 88% | 94% | 89% |
| **negative predictive value (NPV)** | 91% | 89% | 88% | 87% |
| **positive predictive value (PPV)** | 60% | 39% | 60% | 46% |

**Table 11: Genes included in the gene sets that are able to predict risk of recurrence and/or metastasis**

| **Gene name** | **Probe Identifier (ThermoFisher)** | **median Recurrent** | **median Non-Recurrent** | **delta median** * | **p-value** |
|---|---|---|---|---|---|
| KRT6B | Hs00745492_s1 | 5.522 | 7.091 | -1.569 | 0.000070 |
| LOR | Hs01894962_s1 | 1.970 | 4.492 | -2.522 | 0.000265 |
| FLG | Hs00856927_g1 | -2.724 | 0.303 | -3.027 | 0.000291 |
| LCE2B | Hs04194422_s1 | 1.153 | 3.665 | -2.512 | 0.000809 |
| PLS3 | Hs00543973_m1 | -0.416 | 0.080 | -0.497 | 0.001048 |
| SERPINB2 | Hs01010736_m1 | 0.304 | 1.455 | -1.150 | 0.001277 |
| KLK5 | Hs00202752_m1 | 1.170 | 3.239 | -2.069 | 0.001468 |
| KRT18 | Hs01920599_gH | 0.975 | -0.238 | 1.213 | 0.002094 |
| BBC | Hs00248075_m1 | -4.614 | -5.334 | 0.720 | 0.002663 |
| MIR3916 | Hs04232205_s1 | -0.709 | -1.334 | 0.625 | 0.002734 |
| LOC100287896 | Hs01931732_s1 | -2.224 | -2.796 | 0.572 | 0.003547 |
| TFAP2B | Hs01560931_m1 | -4.288 | -2.456 | -1.832 | 0.004135 |
| HPGD | Hs00960591_m1 | -5.491 | -3.113 | -2.378 | 0.007656 |
| CHMP2B | Hs00387770_m1 | -3.117 | -2.591 | -0.526 | 0.008827 |
| ANXA9 | Hs01070154_m1 | -5.583 | -4.284 | -1.299 | 0.009038 |
| ID2 | Hs00747379_m1 | -0.345 | 0.493 | -0.838 | 0.009695 |
| EphB2 | Hs00362096_m1 | -1.124 | -1.614 | 0.491 | 0.012203 |
| NEB | Hs00189880_m1 | -2.611 | -1.904 | -0.706 | 0.014937 |
| FDFT1 | Hs00926053_m1 | -1.589 | -0.657 | -0.932 | 0.017046 |
| USP7 | Hs00931763_m1 | 1.509 | 1.960 | -0.452 | 0.017046 |
| TAF6L | Hs01008033_m1 | -0.699 | -0.961 | 0.262 | 0.018195 |
| ACSBG1 | Hs01025572_m1 | -2.992 | -1.336 | -1.657 | 0.026077 |
| HNRNPL | Hs00704853_s1 | 0.776 | 0.980 | -0.204 | 0.031337 |
| ARPC2 | Hs01031740_m1 | 0.715 | 1.147 | -0.432 | 0.031337 |
| DUXAP8 | Hs04942686_m1 | -6.816 | -9.507 | 2.691 | 0.039746 |
| PIM2 | Hs01546752_g1 | -1.160 | -1.752 | 0.592 | 0.050944 |
| KRT17 | Hs00356958_m1 | 6.944 | 7.254 | -0.310 | 0.053874 |
| APOBEC3G | Hs00222415_m1 | -2.574 | -3.024 | 0.450 | 0.056942 |
| DSS1 | Hs00428732_m1 | -4.131 | -3.182 | -0.949 | 0.056942 |
| EGFR | Hs01076090_m1 | 1.598 | 1.332 | 0.266 | 0.069464 |
| SERPINB4 | Hs01691258_g1 | -12.838 | -8.116 | -4.722 | 0.070706 |
| UGP2 | Hs00900510_m1 | -1.783 | -1.437 | -0.346 | 0.073246 |
| SPATA41 | Hs03028557_s1 | -12.073 | -13.333 | 1.261 | 0.077195 |
| SNORD124 | Hs03464469_s1 | -2.848 | -2.958 | 0.110 | 0.082729 |
| PI3 | Hs00964384_g1 | 5.550 | 6.140 | -0.589 | 0.085614 |
| LIME1-ZGPAT | Hs00738791_g1 | -4.044 | -4.312 | 0.267 | 0.090094 |
| MMP3 | Hs00968305_m1 | -1.478 | -2.397 | 0.919 | 0.099619 |
| S100A8 | Hs00374264_g1 | 4.237 | 5.014 | -0.777 | 0.104673 |
| PTRHD1 | Hs00415546_m1 | -1.338 | -1.216 | -0.122 | 0.109930 |
| MMP7 | Hs01042796 ml | -2.399 | -3.937 | 1.538 | 0.115392 |
| TMEM41B | Hs01379134_m1 | -1.979 | -1.562 | -0.417 | 0.119151 |
| SPP1 | Hs00959010_m1 | 1.650 | 0.427 | 1.224 | 0.121066 |
| RBM33 | Hs00997579_m1 | 1.600 | 1.349 | 0.251 | 0.152768 |
| NFIB | Hs01029174 ml | -1.757 | -1.633 | -0.124 | 0.159806 |
| NEFL | Hs00196245_m1 | -0.069 | 0.561 | -0.631 | 0.162206 |
| NFIC | Hs00232157_m1 | -0.500 | -0.300 | -0.200 | 0.167086 |
| DCT | Hs01098278_m1 | -3.033 | -1.300 | -1.733 | 0.174613 |
| RCHY1 | Hs00996236_m1 | -1.807 | -2.038 | 0.231 | 0.177178 |
| ZSCAN31 | Hs00372831_g1 | -3.639 | -2.926 | -0.713 | 0.179770 |
| IPO5P1 | Hs05052601_s1 | -2.927 | -3.231 | 0.303 | 0.179770 |
| RUNX3 | Hs00231709_m1 | -0.927 | -1.342 | 0.415 | 0.204381 |
| MKLN1 | Hs00992679_m1 | -0.787 | -0.930 | 0.144 | 0.204381 |
| ATP6V0E2 | Hs04189864_m1 | -5.596 | -6.247 | 0.651 | 0.207260 |
| YKT6 | Hs00559914_m1 | 2.007 | 1.788 | 0.220 | 0.210168 |
| FCHSD1 | Hs00703025_s1 | -6.048 | -5.195 | -0.854 | 0.216073 |
| MMP 1 | Hs00899658_m1 | 3.156 | 2.381 | 0.774 | 0.225153 |
| CEP76 | Hs00950371_m1 | -3.743 | -3.455 | -0.288 | 0.225153 |
| TUFM-MIR4721 | Hs00944507_g1 | 2.465 | 2.281 | 0.184 | 0.228239 |
| AIM2 | Hs00915710_m1 | -2.525 | -2.720 | 0.195 | 0.244123 |
| PTHLH | Hs00174969_m1 | 0.986 | 1.833 | -0.848 | 0.264188 |
| BHLHB9 | Hs01089557_s1 | -14.090 | -12.657 | -1.433 | 0.264188 |
| CD 163 | Hs00174705_m1 | -0.829 | -1.156 | 0.327 | 0.307655 |
| ZNF839 | Hs00901350_g1 | -1.060 | -1.316 | 0.256 | 0.307655 |
| BLOC1S1 | Hs00155241_m1 | -1.061 | -0.787 | -0.273 | 0.311480 |
| HDDC3 | Hs00826827_g1 | -1.299 | -1.567 | 0.267 | 0.319223 |
| TNNC1 | Hs00896999_g1 | -7.015 | -5.911 | -1.105 | 0.323141 |
| S100A9 | Hs00610058_m1 | 8.071 | 8.385 | -0.314 | 0.327091 |
| TUBB3 | Hs00801390_s1 | -3.190 | -2.711 | -0.479 | 0.331071 |
| KIT | Hs00174029_m1 | -1.168 | -1.574 | 0.406 | 0.351443 |
| FN1 | Hs01549976_m1 | 2.302 | 1.859 | 0.443 | 0.364039 |
| INHBA | Hs01081598_m1 | -1.107 | -1.166 | 0.060 | 0.368299 |
| PIGBOS1 | Hs05036222_s1 | -0.970 | -1.132 | 0.162 | 0.372591 |
| THYN1 | Hs01553775_g1 | 0.011 | -0.219 | 0.230 | 0.376913 |
| HOXA10-HOXA9-MIR196B | Hs00365956_m1 | -3.574 | -2.521 | -1.053 | 0.412594 |
| MYC | Hs00153408_m1 | -2.553 | -2.337 | -0.215 | 0.440624 |
| IL24 | Hs01114274_m1 | -3.039 | -3.394 | 0.355 | 0.455038 |
| NFIA | Hs00379134_m1 | -0.852 | -0.709 | -0.143 | 0.499836 |
| RPL26L1 | Hs01631495_s1 | -6.405 | -6.603 | 0.198 | 0.504954 |
| ZNF48 | Hs00399035_m1 | -3.340 | -3.577 | 0.237 | 0.520473 |
| MIER2 | Hs00380101_m1 | -0.275 | -0.382 | 0.108 | 0.530953 |
| MMP13 | Hs00942584_m1 | -4.547 | -5.058 | 0.511 | 0.536233 |
| TYRP1 | Hs00167051_m1 | -2.547 | -2.510 | -0.037 | 0.546872 |
| VIM | Hs00958111_m1 | 4.763 | 4.373 | 0.390 | 0.552231 |
| LRRC47 | Hs00975850_m1 | 0.130 | 0.070 | 0.060 | 0.552231 |
| ALOX 12 | Hs00167524_m1 | -3.032 | -2.563 | -0.469 | 0.590445 |
| PLAU | Hs01547054_m1 | 3.212 | 2.870 | 0.342 | 0.612814 |
| IL7R | Hs00902334_m1 | -4.480 | -4.820 | 0.340 | 0.624137 |
| DARS | Hs00962398_m1 | 2.314 | 2.486 | -0.172 | 0.624137 |
| LOC101927502 | Hs05033260_s1 | -8.529 | -8.227 | -0.302 | 0.624137 |
| MIR129-1 | Hs03302824_pri | -12.122 | -13.033 | 0.910 | 0.647050 |
| PD1 | Hs00240906_m1 | -1.233 | -1.099 | -0.134 | 0.652832 |
| CYP2D6-LOC101929829 | Hs03043789_g1 | -5.343 | -5.128 | -0.215 | 0.676166 |
| GTPBP2 | Hs01051445_g1 | -2.289 | -2.127 | -0.163 | 0.687952 |
| CXCL 10 | Hs00171042_m1 | -1.850 | -1.595 | -0.255 | 0.693874 |
| SLC1A3 | Hs00904817_m1 | -2.518 | -2.534 | 0.016 | 0.699815 |
| RNF 135 | Hs00260480_m1 | -0.694 | -0.725 | 0.030 | 0.711752 |
| NOA1 | Hs00260452_m1 | -2.426 | -2.528 | 0.102 | 0.747977 |
| ZNF496 | Hs00262107_m1 | -1.484 | -1.549 | 0.065 | 0.760181 |
| MMP12 | Hs00159178_m1 | -2.301 | -2.567 | 0.266 | 0.772445 |
| C3orf70 | Hs01395177_m1 | -4.175 | -4.227 | 0.052 | 0.784767 |
| LAMC2 | Hs01043717_m1 | 0.874 | 1.000 | -0.126 | 0.797143 |
| MMP10 | Hs00233987_m1 | -0.255 | 0.441 | -0.696 | 0.803350 |
| C1QL4 | Hs00884853_s1 | -10.397 | -10.511 | 0.113 | 0.822045 |
| C21orf59 | Hs00937509_m1 | 0.903 | 0.829 | 0.074 | 0.822045 |
| KRT19 | Hs01051611_gH | -3.414 | -2.626 | -0.788 | 0.828299 |
| PDL1 | Hs00204257_m1 | -2.051 | -2.218 | 0.166 | 0.847127 |
| SLC25A11 | Hs01087946_g1 | 0.664 | 0.641 | 0.024 | 0.847127 |
| MRC1 | Hs00267207_m1 | -5.005 | -5.020 | 0.015 | 0.853423 |
| PIG3 | Hs00936519_m1 | -3.104 | -2.896 | -0.207 | 0.853423 |
| IL2RB | Hs00386692_m1 | -1.702 | -1.592 | -0.110 | 0.878697 |
| ATP6AP1 | Hs05016463_s1 | 0.173 | 0.183 | -0.011 | 0.878697 |
| MSANTD4 | Hs00411188_gl | -3.627 | -3.612 | -0.015 | 0.929591 |
| MRPL21 | Hs00698959_m1 | 0.665 | 0.664 | 0.002 | 0.929591 |
| CXCR4 | Hs00607978_s1 | -5.555 | -5.868 | 0.313 | 0.935977 |
| RPP38 | Hs00705626_s1 | -4.839 | -4.719 | -0.120 | 0.935977 |
| SEPT3 | Hs00251883_m1 | -5.165 | -5.094 | -0.071 | 0.942368 |
| PDPN | Hs00366766_m1 | 1.995 | 1.893 | 0.102 | 0.948762 |
| CCL27 | Hs00171157_m1 | -12.962 | -11.407 | -1.555 | 0.967963 |
| CHI3L1 | Hs01072228_m1 | 0.794 | 0.689 | 0.105 | 0.974368 |
| DDAH1 | Hs00201707_m1 | -3.775 | -3.568 | -0.207 | 0.980774 |
| MMP9 | Hs00957562_m1 | 2.233 | 2.368 | -0.135 | 0.987182 |
| NFASC | Hs00978280_m1 | -2.781 | -2.716 | -0.066 | 0.993591 |
| * Positive values indicate an INCREASE in gene expression in recurrent cancer when compared to non-recurrent control; and negative values indicate a DECREASE in gene expression in recurrent cancer when compared to non-recurrent control. | | | | | |

**Table 12: Accuracy of gene sets used to predict risk of recurrence and/or metastasis**

| **Gene set** | **Sensitivity** | **Specificity** | **PPV** | **NPV** | **AUC** | **Kappa** |
|---|---|---|---|---|---|---|
| 20-1 | 0.4958 | 0.9481 | 0.5931 | 0.9370 | 0.8604 | 0.4537 |
| 20-2 | 0.5208 | 0.9333 | 0.5869 | 0.9385 | 0.8101 | 0.4524 |
| 20-3 | 0.4438 | 0.9472 | 0.5829 | 0.9292 | 0.8131 | 0.4147 |
| 20-4 | 0.4708 | 0.9324 | 0.5688 | 0.9318 | 0.8766 | 0.4084 |
| 20-5 | 0.4833 | 0.9324 | 0.4990 | 0.9335 | 0.8242 | 0.4033 |
| 20-6 | 0.4542 | 0.9389 | 0.5722 | 0.9306 | 0.8275 | 0.3991 |
| 20-7 | 0.5396 | 0.9065 | 0.4634 | 0.9395 | 0.8384 | 0.3934 |
| 20-8 | 0.3917 | 0.9537 | 0.5922 | 0.9238 | 0.7275 | 0.3783 |
| 20-9 | 0.4396 | 0.9259 | 0.5132 | 0.9274 | 0.8220 | 0.3673 |
| 20-10 | 0.3708 | 0.9556 | 0.5888 | 0.9228 | 0.7970 | 0.3621 |
| 20-11 | 0.4542 | 0.9241 | 0.4625 | 0.9299 | 0.7701 | 0.3615 |
| 20-12 | 0.4292 | 0.9324 | 0.4984 | 0.9280 | 0.7876 | 0.3613 |
| 20-13 | 0.3896 | 0.9472 | 0.5367 | 0.9234 | 0.8228 | 0.3605 |
| 20-14 | 0.4146 | 0.9343 | 0.5150 | 0.9254 | 0.7698 | 0.3600 |
| 20-15 | 0.4417 | 0.9278 | 0.4798 | 0.9299 | 0.7799 | 0.3553 |
| 20-16 | 0.4271 | 0.9278 | 0.4667 | 0.9262 | 0.7650 | 0.3506 |
| 20-17 | 0.4146 | 0.9287 | 0.4673 | 0.9248 | 0.7613 | 0.3506 |
| 20-18 | 0.4563 | 0.9139 | 0.4518 | 0.9297 | 0.8198 | 0.3491 |
| 20-19 | 0.4188 | 0.9315 | 0.5352 | 0.9270 | 0.8132 | 0.3489 |
| 20-20 | 0.4229 | 0.9296 | 0.4484 | 0.9264 | 0.7674 | 0.3438 |
| 20-21 | 0.4396 | 0.9231 | 0.4449 | 0.9290 | 0.8336 | 0.3420 |
| 20-22 | 0.4354 | 0.9194 | 0.4282 | 0.9268 | 0.8127 | 0.3418 |
| 20-23 | 0.3563 | 0.9537 | 0.5608 | 0.9213 | 0.7605 | 0.3379 |
| 20-24 | 0.3896 | 0.9296 | 0.4846 | 0.9221 | 0.7662 | 0.3357 |
| 20-25 | 0.3896 | 0.9370 | 0.4919 | 0.9218 | 0.8326 | 0.3354 |
| 30-1 | 0.4021 | 0.9648 | 0.6285 | 0.9275 | 0.8091 | 0.3893 |
| 30-2 | 0.4771 | 0.9204 | 0.4672 | 0.9335 | 0.8005 | 0.3739 |
| 30-3 | 0.4438 | 0.9287 | 0.4984 | 0.9287 | 0.8083 | 0.3685 |
| 30-4 | 0.4208 | 0.9306 | 0.5525 | 0.9270 | 0.8064 | 0.3613 |
| 30-5 | 0.4000 | 0.9407 | 0.5432 | 0.9255 | 0.7804 | 0.3513 |
| 30-6 | 0.4542 | 0.9167 | 0.4574 | 0.9288 | 0.7920 | 0.3480 |
| 30-7 | 0.3875 | 0.9407 | 0.5049 | 0.9240 | 0.8209 | 0.3378 |
| 30-8 | 0.3792 | 0.9454 | 0.5218 | 0.9225 | 0.7739 | 0.3375 |
| 30-9 | 0.3542 | 0.9593 | 0.5714 | 0.9207 | 0.6822 | 0.3347 |
| 30-10 | 0.4458 | 0.9157 | 0.4271 | 0.9281 | 0.7544 | 0.3339 |
| 30-11 | 0.4167 | 0.9213 | 0.4288 | 0.9245 | 0.7915 | 0.3329 |
| 30-12 | 0.3813 | 0.9380 | 0.4732 | 0.9216 | 0.7236 | 0.3318 |
| 30-13 | 0.3229 | 0.9565 | 0.6146 | 0.9170 | 0.7093 | 0.3243 |
| 30-14 | 0.3729 | 0.9361 | 0.4768 | 0.9222 | 0.7127 | 0.3187 |
| 30-15 | 0.4042 | 0.9176 | 0.3988 | 0.9225 | 0.7716 | 0.3103 |
| 30-16 | 0.3667 | 0.9306 | 0.4688 | 0.9195 | 0.7127 | 0.3052 |
| 30-17 | 0.3375 | 0.9426 | 0.4744 | 0.9178 | 0.6708 | 0.3025 |
| 30-18 | 0.3813 | 0.9213 | 0.4323 | 0.9205 | 0.8029 | 0.2996 |
| 30-19 | 0.4146 | 0.9102 | 0.3787 | 0.9241 | 0.7652 | 0.2984 |
| 30-20 | 0.3667 | 0.9296 | 0.4427 | 0.9199 | 0.7452 | 0.2954 |
| 30-21 | 0.3583 | 0.9306 | 0.4480 | 0.9197 | 0.7475 | 0.2900 |
| 30-22 | 0.3625 | 0.9241 | 0.4685 | 0.9181 | 0.7671 | 0.2897 |
| 30-23 | 0.3833 | 0.9194 | 0.3956 | 0.9199 | 0.7480 | 0.2891 |
| 30-24 | 0.3417 | 0.9380 | 0.4473 | 0.9187 | 0.7222 | 0.2885 |
| 30-25 | 0.3979 | 0.9120 | 0.3898 | 0.9221 | 0.7419 | 0.2868 |
| 40-1 | 0.4688 | 0.9481 | 0.6105 | 0.9334 | 0.8198 | 0.4340 |
| 40-2 | 0.4021 | 0.9435 | 0.5360 | 0.9242 | 0.7960 | 0.3565 |
| 40-3 | 0.3792 | 0.9426 | 0.5311 | 0.9230 | 0.7486 | 0.3354 |
| 40-4 | 0.3563 | 0.9509 | 0.5030 | 0.9200 | 0.7427 | 0.3325 |
| 40-5 | 0.4125 | 0.9278 | 0.4898 | 0.9257 | 0.8127 | 0.3300 |
| 40-6 | 0.3896 | 0.9361 | 0.4924 | 0.9235 | 0.7824 | 0.3294 |
| 40-7 | 0.3854 | 0.9324 | 0.4662 | 0.9219 | 0.7421 | 0.3248 |
| 40-8 | 0.3646 | 0.9398 | 0.5220 | 0.9212 | 0.7262 | 0.3228 |
| 40-9 | 0.3583 | 0.9380 | 0.5303 | 0.9199 | 0.7621 | 0.3189 |
| 40-10 | 0.3500 | 0.9472 | 0.4906 | 0.9201 | 0.7059 | 0.3161 |
| 40-11 | 0.3938 | 0.9222 | 0.4707 | 0.9225 | 0.7623 | 0.3143 |
| 40-12 | 0.3417 | 0.9500 | 0.5070 | 0.9188 | 0.7769 | 0.3115 |
| 40-13 | 0.3896 | 0.9296 | 0.4195 | 0.9233 | 0.7851 | 0.3047 |
| 40-14 | 0.3479 | 0.9407 | 0.4750 | 0.9178 | 0.8177 | 0.3036 |
| 40-15 | 0.3729 | 0.9269 | 0.4124 | 0.9206 | 0.6769 | 0.3034 |
| 40-16 | 0.3646 | 0.9343 | 0.4224 | 0.9200 | 0.7467 | 0.2989 |
| 40-17 | 0.3792 | 0.9222 | 0.4296 | 0.9204 | 0.7539 | 0.2983 |
| 40-18 | 0.3208 | 0.9435 | 0.5381 | 0.9152 | 0.6774 | 0.2980 |
| 40-19 | 0.3688 | 0.9194 | 0.4660 | 0.9192 | 0.6873 | 0.2940 |
| 40-20 | 0.3854 | 0.9204 | 0.4162 | 0.9224 | 0.8275 | 0.2939 |
| 40-21 | 0.3833 | 0.9167 | 0.3896 | 0.9215 | 0.7007 | 0.2904 |
| 40-22 | 0.3625 | 0.9185 | 0.4270 | 0.9177 | 0.6769 | 0.2904 |
| 40-23 | 0.3313 | 0.9343 | 0.4227 | 0.9160 | 0.6716 | 0.2836 |
| 40-24 | 0.3438 | 0.9407 | 0.4236 | 0.9193 | 0.6736 | 0.2799 |
| 40-25 | 0.3250 | 0.9389 | 0.4582 | 0.9160 | 0.6687 | 0.2774 |

**Table 13: Exemplary gene sets used to predict risk of recurrence and/or metastasis**

| **Gene set** | **Probe identifiers used for each gene set (probe identifiers from ThermoFisher Scientific).** |
|---|---|
| 20-1 | "Hs00705626_s1" "Hs00248075_m1" "Hs01560931_m1" "Hs00167524_m1" |
| | "Hs00366766_m1" "Hs01051445_g1" "Hs00996236_m1" "Hs01089557_s1" |
| | "Hs00262107_m1" "Hs01931732_s1" "Hs00399035_m1" "Hs00231709_m1" |
| | "Hs00411188_g1" "Hs00978280_m1" "Hs00826827_g1" "Hs00232157_m1" |
| | "Hs00747379_m1" "Hs00233987_m1" "Hs01008033_m1" "Hs04194422_s1" |
| 20-2 | "Hs00884853_s1" "Hs01920599_gH" "Hs00996236_m1" "Hs00248075_m1" |
| | "Hs00167524_m1" "Hs00747379_m1" "Hs00942584_m1" "Hs01042796_m1" |
| | "Hs00964384_g1" "Hs05052601_s1" "Hs00356958_m1" "Hs00901350_g1" |
| | "Hs01691258_g1" "Hs00992679_m1" "Hs01051611_gH" "Hs04194422_s1" |
| | "Hs01089557_s1" "Hs01087946_g1" "Hs05036222_s1" "Hs00856927_g1" |
| 20-3 | "Hs00248075_m1" "Hs00251883_m1" "Hs01089557_s1" "Hs00356958_m1" |
| | "Hs00856927_g1" "Hs00202752_m1" "Hs00950371_m1" "Hs00899658_m1" |
| | "Hs00362096_m1" "Hs01043717_m1" "Hs01560931_m1" "Hs00826827_g1" |
| | "Hs01010736_m1" "Hs00167524_m1" "Hs01031740_m1" "Hs01920599_gH" |
| | "Hs00201707_m1" "Hs00738791_g1" "Hs00962398_m1" "Hs00543973_m1" |
| 20-4 | "Hs00962398_m1" "Hs01920599_gH" "Hs01025572_m1" "Hs00159178_m1" |
| | "Hs01089557_s1" "Hs00167524_m1" "Hs00248075_m1" "Hs00386692_m1" |
| | "Hs00856927_g1" "Hs00996236_m1" "Hs01031740_m1" "Hs01010736_m1" |
| | "Hs00900510_m1" "Hs00826827_g1" "Hs01008033_m1" "Hs00415546_m1" |
| | "Hs04942686_m1" "Hs00801390_s1" "Hs01072228_m1" "Hs01547054_m1" |
| 20-5 | "Hs00411188_g1" "Hs00248075_m1" "Hs00202752_m1" "Hs00747379_m1" |
| | "Hs01042796_m1" "Hs01920599_gH" "Hs01114274_m1" "Hs00942584_m1" |
| | "Hs00996236_m1" "Hs00167524_m1" "Hs00978280_m1" "Hs00543973_m1" |
| | "Hs00826827_g1" "Hs01560931_m1" "Hs00931763_m1" "Hs01089557_s1" |
| | "Hs00174029_m1" "Hs01029174_m1" "Hs00415546_m1" "Hs00964384_g1" |
| 20-6 | "Hs00975850_m1" "Hs04942686_m1" "Hs00202752_m1" "Hs00233987_m1" |
| | "Hs00926053_m1" "Hs00856927_g1" "Hs00992679_m1" "Hs00251883_m1" |
| | "Hs00415546_m1" "Hs00960591_m1" "Hs00901350_g1" "Hs00747379_m1" |
| | "Hs01089557_s1" "Hs00936519_m1" "Hs03043789_g1" "Hs01560931_m1" |
| | "Hs00232157_m1" "Hs00957562_m1" "Hs00248075_m1" "Hs01549976_m1" |
| 20-7 | "Hs04194422_s1" "Hs00262107_m1" "Hs01546752_g1" "Hs01920599_gH" |
| | "Hs04189864_m1" "Hs01089557_s1" "Hs01560931_m1" "Hs00705626_s1" |
| | "Hs01043717_m1" "Hs00747379_m1" "Hs00248075_m1" "Hs00856927_g1" |
| | "Hs01029174_m1" "Hs00543973_m1" "Hs01395177_m1" "Hs00260480_m1" |
| | "Hs00174029_m1" "Hs00387770_m1" "Hs01894962_s1" "Hs00745492_s1" |
| 20-8 | "Hs01560931_m1" "Hs00738791_g1" "Hs00856927_g1" "Hs00362096_m1" |
| | "Hs00826827_g1" "Hs01098278_m1" "Hs00975850_m1" "Hs00167524_m1" |
| | "Hs00260452_m1" "Hs04194422_s1" "Hs01043717_m1" "Hs00233987_m1" |
| | "Hs00703025_s1" "Hs00896999_g1" "Hs00167051_m1" "Hs00942584_m1" |
| | "Hs01087946_g1" "Hs00411188_g1" "Hs00747379_m1" "Hs05016463_s1" |
| 20-9 | "Hs00362096_m1" "Hs00942584_m1" "Hs01560931_m1" "Hs00167524_m1" |
| | "Hs00884853_s1" "Hs00248075_m1" "Hs01920599_gH" "Hs00996236_m1" |
| | "Hs00747379_m1" "Hs01089557_s1" "Hs00959010_m1" "Hs00372831_gl" |
| | "Hs04194422_s1" "Hs01043717_m1" "Hs00399035_m1" "Hs01051611_gH" |
| | "Hs01042796_m1" "Hs00968305_m1" "Hs00260452_m1" "Hs01031740_m1" |
| 20-10 | "Hs05033260_s1" "Hs00233987_m1" "Hs04194422_s1" "Hs00992679_m1" |
| | "Hs00926053_m1" "Hs00167524_m1" "Hs00202752_m1" "Hs01549976_m1" |
| | "Hs00415546_m1" "Hs01072228_m1" "Hs01691258_g1" "Hs00387770_m1" |
| | "Hs00380101_m1" "Hs00231709_m1" "Hs01920599_gH" "Hs00543973_m1" |
| | "Hs00386692_m1" "Hs00705626_s1" "Hs00196245_m1" "Hs01081598_m1" |
| 20-11 | "Hs01114274_m1" "Hs01560931_m1" "Hs00738791_g1" "Hs00931763_m1" |
| | "Hs00996236_m1" "Hs00362096_m1" "Hs00747379_m1" "Hs00411188_g1" |
| | "Hs00900510_m1" "Hs01098278_m1" "Hs00233987_m1" "Hs04194422_s1" |
| | "Hs00826827_g1" "Hs00856927_g1" "Hs00232157_m1" "Hs01010736_m1" |
| | "Hs00704853_s1" "Hs00959010_m1" "Hs00260480_m1" "Hs00915710_m1" |
| 20-12 | "Hs00992679_m1" "Hs00159178_m1" "Hs00167524_m1" "Hs00958111_m1" |
| | "Hs00901350_g1" "Hs00931763_m1" "Hs00233987_m1" "Hs01549976_m1" |
| | "Hs01894962_s1" "Hs01089557_s1" "Hs00171157_m1" "Hs00153408_m1" |
| | "Hs00248075_m1" "Hs03464469_s1" "Hs04194422_s1" "Hs00745492_s1" |
| | "Hs00366766_m1" "Hs00856927_g1" "Hs00957562_m1" "Hs01025572_m1" |
| 20-13 | "Hs01379134_m1" "Hs00362096_m1" "Hs05052601_s1" "Hs00959010_m1" |
| | "Hs00251883_m1" "Hs01089557_s1" "Hs00856927_g1" "Hs00167524_m1" |
| | "Hs00159178_m1" "Hs04942686_m1" "Hs00356958_m1" "Hs01042796_m1" |
| | "Hs03464469_s1" "Hs01029174_m1" "Hs00248075_m1" "Hs00610058_m1" |
| | "Hs01070154_m1" "Hs00703025_s1" "Hs00964384_g1" "Hs00705626_s1" |
| 20-14 | "Hs00362096_m1" "Hs00996236_m1" "Hs00931763_m1" "Hs01081598_m1" |
| | "Hs01560931_m1" "Hs00167524_m1" "Hs00543973_m1" "Hs01098278_m1" |
| | "Hs00856927_g1" "Hs03043789_g1" "Hs01089557_s1" "Hs01051445_g1" |
| | "Hs00747379_m1" "Hs01114274_m1" "Hs00826827_g1" "Hs00936519_m1" |
| | "Hs00960591_m1" "Hs00201707_m1" "Hs00899658_m1" "Hs04189864_m1" |
| 20-15 | "Hs00856927_g1" "Hs04942686_m1" "Hs00248075_m1" "Hs01029174_m1" |
| | "Hs00992679_m1" "Hs00975850_m1" "Hs03464469_s1" "Hs00167524_m1" |
| | "Hs01691258_g1" "Hs00960591_m1" "Hs01089557_s1" "Hs04194422_s1" |
| | "Hs01114274_m1" "Hs00901350_g1" "Hs00936519_m1" "Hs00233987_m1" |
| | "Hs00411188_g1" "Hs00900510_m1" "Hs00174969_m1" "Hs01070154_m1" |
| 20-16 | "Hs00826827_g1" "Hs00738791_g1" "Hs00698959_m1" "Hs00153408_m1" |
| | "Hs00167051_m1" "Hs00365956_m1" "Hs04194422_s1" "Hs00167524_m1" |
| | "Hs01560931_m1" "Hs00610058_m1" "Hs00232157_m1" "Hs00996236_m1" |
| | "Hs00705626_s1" "Hs00362096_m1" "Hs01098278_m1" "Hs00997579_m1" |
| | "Hs00559914_m1" "Hs00856927_g1" "Hs00944507_g1" "Hs00379134_m1" |
| 20-17 | "Hs00232157_m1" "Hs00543973_m1" "Hs05052601_s1" "Hs00196245_m1" |
| | "Hs01042796_m1" "Hs00411188_g1" "Hs00899658_m1" "Hs00374264_g1" |
| | "Hs01894962_s1" "Hs04194422_s1" "Hs03464469_s1" "Hs00992679_m1" |
| | "Hs00901350_g1" "Hs00362096_m1" "Hs00856927_g1" "Hs00167524_m1" |
| | "Hs01089557_s1" "Hs01931732_s1" "Hs01549976_m1" "Hs01395177_m1" |
| 20-18 | "Hs00884853_s1" "Hs00167524_m1" "Hs00978280_m1" "Hs00747379_m1" |
| | "Hs01931732_s1" "Hs00931763_m1" "Hs04942686_m1" "Hs04194422_s1" |
| | "Hs00856927_g1" "Hs00248075_m1" "Hs00901350_g1" "Hs00415546_m1" |
| | "Hs01089557_s1" "Hs01025572_m1" "Hs00231709_m1" "Hs00386692_m1" |
| | "Hs01920599_gH" "Hs00202752_m1" "Hs01029174_m1" "Hs00942584_m1" |
| 20-19 | "Hs01547054_m1" "Hs00960591_m1" "Hs03464469_s1" "Hs01560931_m1" |
| | "Hs00153408_m1" "Hs00233987_m1" "Hs01089557_s1" "Hs00366766_m1" |
| | "Hs00248075_m1" "Hs01010736_m1" "Hs00251883_m1" "Hs00996236_m1" |
| | "Hs00610058_m1" "Hs00900510_m1" "Hs01008033_m1" "Hs00978280_m1" |
| | "Hs00260452_m1" "Hs04194422_s1" "Hs00196245_m1" "Hs05052601_s1" |
| 20-20 | "Hs01098278_m1" "Hs01072228_m1" "Hs01691258_g1" "Hs00387770_m1" |
| | "Hs00543973_m1" "Hs01920599_gH" "Hs04189864_m1" "Hs05033260_s1" |
| | "Hs00856927_g1" "Hs00366766_m1" "Hs03043789_g1" "Hs04194422_s1" |
| | "Hs00202752_m1" "Hs00936519_m1" "Hs01560931_m1" "Hs00958111_m1" |
| | "Hs00251883_m1" "Hs00704853_s1" "Hs00738791_g1" "Hs00962398_m1" |
| 20-21 | "Hs01042796_m1" "Hs01560931_m1" "Hs00884853_s1" "Hs00958111_m1" |
| | "Hs00411188_g1" "Hs05052601_s1" "Hs01920599_gH" "Hs00248075_m1" |
| | "Hs00747379_m1" "Hs00996236_m1" "Hs00251883_m1" "Hs00202752_m1" |
| | "Hs01008033_m1" "Hs00201707_m1" "Hs01051445_g1" "Hs00950371_m1" |
| | "Hs01029174_m1" "Hs00232157_m1" "Hs01087946_g1" "Hs00267207_m1" |
| 20-22 | "Hs00559914_m1" "Hs00856927_g1" "Hs00978280_m1" "Hs01920599_gH" |
| | "Hs01560931_m1" "Hs00365956_m1" "Hs00610058_m1" "Hs01008033_m1" |
| | "Hs04194422_s1" "Hs00975850_m1" "Hs00204257_m1" "Hs00950371_m1" |
| | "Hs00705626_s1" "Hs01089557_s1" "Hs00196245_m1" "Hs01042796_m1" |
| | "Hs00174029_m1" "Hs01546752_g1" "Hs01098278_m1" "Hs00231709_m1" |
| 20-23 | "Hs04194422_s1" "Hs01089557_s1" "Hs00155241_m1" "Hs00942584_m1" |
| | "Hs05033260_s1" "Hs01546752_g1" "Hs01920599_gH" "Hs01560931_m1" |
| | "Hs00387770_m1" "Hs00399035_m1" "Hs00232157_m1" "Hs00931763_m1" |
| | "Hs00231709_m1" "Hs00856927_g1" "Hs00233987_m1" "Hs01098278_m1" |
| | "Hs00978280_m1" "Hs04942686_m1" "Hs00411188_g1" "Hs00201707_m1" |
| 20-24 | "Hs00232157_m1" "Hs01010736_m1" "Hs00704853_s1" "Hs00167051_m1" |
| | "Hs00738791_g1" "Hs00975850_m1" "Hs00362096_m1" "Hs00826827_g1" |
| | "Hs00233987_m1" "Hs00950371_m1" "Hs00204257_m1" "Hs01560931_m1" |
| | "Hs00196245_m1" "Hs01042796_m1" "Hs00174029_m1" "Hs00705626_s1" |
| | "Hs00856927_g1" "Hs01051445_g1" "Hs00399035_m1" "Hs05036222_s1" |
| 20-25 | "Hs00233987_m1" "Hs00380101_m1" "Hs00167524_m1" "Hs04189864_m1" |
| | "Hs00543973_m1" "Hs01920599_gH" "Hs00856927_g1" "Hs00155241_m1" |
| | "Hs01098278_m1" "Hs00703025_s1" "Hs00231709_m1" "Hs00365956_m1" |
| | "Hs00204257_m1" "Hs01395177_m1" "Hs00968305_m1" "Hs00374264_g1" |
| | "Hs00248075_m1" "Hs00202752_m1" "Hs00801390_s1" "Hs03464469_s1" |
| 30-1 | "Hs00387770_m1" "Hs00155241_m1" "Hs00251883_m1" "Hs01560931_m1" |
| | "Hs00428732_m1" "Hs00992679_m1" "Hs00705626_s1" "Hs00356958_m1" |
| | "Hs00960591_m1" "Hs00171157_m1" "Hs01072228_m1" "Hs01051445_g1" |
| | "Hs00232157_m1" "Hs00233987_m1" "Hs04232205_s1" "Hs00559914_m1" |
| | "Hs00931763_m1" "Hs00167524_m1" "Hs00171042_m1" "Hs01089557_s1" |
| | "Hs04194422_s1" "Hs03464469_s1" "Hs00944507_g1" "Hs00196245_m1" |
| | "Hs00950371_m1" "Hs01395177_m1" "Hs01070154_m1" "Hs01051611_gH" |
| | "Hs00399035_m1" "Hs00703025_s1" |
| 30-2 | "Hs00937509_m1" "Hs00379134_m1" "Hs00899658_m1" "Hs00248075_m1" |
| | "Hs01920599_gH" "Hs04194422_s1" "Hs03043789_g1" "Hs00610058_m1" |
| | "Hs01089557_s1" "Hs00705626_s1" "Hs00362096_m1" "Hs01560931_m1" |
| | "Hs00233987_m1" "Hs00372831_g1" "Hs01072228_m1" "Hs01379134_m1" |
| | "Hs01076090_m1" "Hs04189864_m1" "Hs00957562_m1" "Hs01931732_s1" |
| | "Hs00856927_g1" "Hs01546752_g1" "Hs00171157_m1" "Hs00992679_m1" |
| | "Hs00931763_m1" "Hs00411188_g1" "Hs01008033_m1" "Hs00387770_m1" |
| | "Hs00996236_m1" "Hs00231709_m1" |
| 30-3 | "Hs00411188_g1" "Hs01395177_m1" "Hs01560931_m1" "Hs00379134_m1" |
| | "Hs00960591_m1" "Hs00745492_s1" "Hs00232157_m1" "Hs00899658_m1" |
| | "Hs00248075_m1" "Hs01010736_m1" "Hs00167524_m1" "Hs01691258_g1" |
| | "Hs00260452_m1" "Hs01070154_m1" "Hs00196245_m1" "Hs00747379_m1" |
| | "Hs01547054_m1" "Hs00950371_m1" "Hs00962398_m1" "Hs01087946_g1" |
| | "Hs00262107_m1" "Hs00260480_m1" "Hs00978280_m1" "Hs00428732_m1" |
| | "Hs00944507_g1" "Hs00387770_m1" "Hs00399035_m1" "Hs00738791_g1" |
| | "Hs00698959_m1" "Hs01549976_m1" |
| 30-4 | "Hs00415546_m1" "Hs00705626_s1" "Hs00204257_m1" "Hs00174969_m1" |
| | "Hs00745492_s1" "Hs01920599_gH" "Hs00387770_m1" "Hs01560931_m1" |
| | "Hs00958111_m1" "Hs01076090_m1" "Hs00379134_m1" "Hs00196245_m1" |
| | "Hs00232157_m1" "Hs01549976_m1" "Hs00801390_s1" "Hs00399035_m1" |
| | "Hs01089557_s1" "Hs00607978_s1" "Hs00960591_m1" "Hs00171157_m1" |
| | "Hs05033260_s1" "Hs00233987_m1" "Hs00904817_m1" "Hs01031740_m1" |
| | "Hs05016463_s1" "Hs00153408_m1" "Hs00968305_m1" "Hs00174029_m1" |
| | "Hs00944507_g1" "Hs00248075_m1" |
| 30-5 | "Hs05033260_s1" "Hs00362096_m1" "Hs00559914_m1" "Hs01560931_m1" |
| | "Hs00968305_m1" "Hs00931763_m1" "Hs04942686_m1" "Hs01920599_gH" |
| | "Hs00899658_m1" "Hs00248075_m1" "Hs00958111_m1" "Hs05016463_s1" |
| | "Hs03464469_s1" "Hs01931732_s1" "Hs01076090_m1" "Hs00992679_m1" |
| | "Hs00901350_g1" "Hs00937509_m1" "Hs00960591_m1" "Hs01114274_m1" |
| | "Hs01043717_m1" "Hs00167524_m1" "Hs01089557_s1" "Hs00204257_m1" |
| | "Hs00703025_s1" "Hs01549976_m1" "Hs01031740_m1" "Hs00262107_m1" |
| | "Hs00957562_m1" "Hs01029174_m1" |
| 30-6 | "Hs00958111_m1" "Hs00411188_g1" "Hs05036222_s1" "Hs00386692_m1" |
| | "Hs00745492_s1" "Hs01010736_m1" "Hs00997579_m1" "Hs00222415_m1" |
| | "Hs03464469_s1" "Hs00233987_m1" "Hs01547054_m1" "Hs00202752_m1" |
| | "Hs01025572_m1" "Hs00379134_m1" "Hs04194422_s1" "Hs00926053_m1" |
| | "Hs01560931_m1" "Hs00705626_s1" "Hs01920599_gH" "Hs00171042_m1" |
| | "Hs03043789_g1" "Hs00610058_m1" "Hs01089557_s1" "Hs00362096_m1" |
| | "Hs00380101_m1" "Hs00899658_m1" "Hs00801390_s1" "Hs00374264_g1" |
| | "Hs00964384_g1" "Hs00240906_m1" |
| 30-7 | "Hs00704853_s1" "Hs01560931_m1" "Hs01379134_m1" "Hs00958111_m1" |
| | "Hs00386692_m1" "Hs04942686_m1" "Hs00747379_m1" "Hs00167524_m1" |
| | "Hs03302824_pri" "Hs00248075_m1" "Hs00387770_m1" "Hs00978280 _m1" |
| | "Hs00379134_m1" "Hs00233987_m1" "Hs00992679_m1" "Hs01931732_s1" |
| | "Hs00380101_m1" "Hs00705626_s1" "Hs00155241_m1" "Hs05033260_s1" |
| | "Hs01089557_s1" "Hs00171157_m1" "Hs00745492_s1" "Hs00937509_m1" |
| | "Hs00374264_g1" "Hs00240906_m1" "Hs01098278_m1" "Hs00174705_m1" |
| | "Hs00260480_m1" "Hs00372831_g1" |
| 30-8 | "Hs00202752_m1" "Hs00231709_m1" "Hs00366766_m1" "Hs00201707_m1" |
| | "Hs00826827_g1" "Hs00856927_g1" "Hs00174969_m1" "Hs00387770_m1" |
| | "Hs00167524_m1" "Hs01031740_m1" "Hs03464469_s1" "Hs01025572_m1" |
| | "Hs00996236_m1" "Hs00950371_m1" "Hs00610058_m1" "Hs01920599_gH" |
| | "Hs01051445_g1" "Hs00975850_m1" "Hs00978280_m1" "Hs00167051_m1" |
| | "Hs01560931 ml" "Hs00704853 _s1" "Hs00902334 _m1" "Hs01553775_g1" |
| | "Hs00962398_m1" "Hs01098278_m1" "Hs00356958_m1" "Hs01087946_g1" |
| | "Hs01029174_m1" "Hs00747379_m1" |
| 30-9 | "Hs00362096_m1" "Hs01560931_m1" "Hs04942686_m1" "Hs05033260_s1" |
| | "Hs00958111_m1" "Hs00232157_m1" "Hs00899658_m1" "Hs00607978_s1" |
| | "Hs01549976_m1" "Hs00262107_m1" "Hs00968305_m1" "Hs00233987_m1" |
| | "Hs00904817_m1" "Hs00356958_m1" "Hs00900510_m1" "Hs00174969_m1" |
| | "Hs04194422_s1" "Hs01043717_m1" "Hs00745492_s1" "Hs00171042_m1" |
| | "Hs00379134_m1" "Hs00559914_m1" "Hs04232205_s1" "Hs00957562_m1" |
| | "Hs00167051_m1" "Hs05052601_s1" "Hs00380101_m1" "Hs01546752_g1" |
| | "Hs00801390_s1" "Hs01070154_m1" |
| 30-10 | "Hs00380101_m1" "Hs03464469_s1" "Hs00372831_g1" "Hs05052601_s1" |
| | "Hs04189864_m1" "Hs00248075_m1" "Hs00968305_m1" "Hs04942686_m1" |
| | "Hs00159178_m1" "Hs00386692_m1" "Hs00937509_m1" "Hs00960591_m1" |
| | "Hs00543973_m1" "Hs01894962_s1" "Hs00233987_m1" "Hs01051445_g1" |
| | "Hs01560931_m1" "Hs01089557_s1" "Hs00232157_m1" "Hs00387770_m1" |
| | "Hs01025572_m1" "Hs01395177_m1" "Hs00801390_s1" "Hs00884853_s1" |
| | "Hs01072228_m1" "Hs00201707_m1" "Hs00996236_m1" "Hs01114274_m1" |
| | "Hs00747379_m1" "Hs00167051_m1" |
| 30-11 | "Hs00801390_s1" "Hs01920599_gH" "Hs00931763_m1" "Hs00262107_m1" |
| | "Hs04942686_m1" "Hs00233987_m1" "Hs00399035_m1" "Hs00248075_m1" |
| | "Hs00171157_m1" "Hs00167524_m1" "Hs00899658_m1" "Hs00232157_m1" |
| | "Hs00362096_m1" "Hs00915710_m1" "Hs00174969_m1" "Hs00900510_m1" |
| | "Hs00387770_m1" "Hs00428732_m1" "Hs00607978_s1" "Hs00380101_m1" |
| | "Hs00260480_m1" "Hs00996236_m1" "Hs00958111_m1" "Hs01070154_m1" |
| | "Hs00543973_m1" "Hs00937509_m1" "Hs01089557_s1" "Hs00968305_m1" |
| | "Hs01072228_m1" "Hs01042796_m1" |
| 30-12 | "Hs00745492_s1" "Hs00950371_m1" "Hs00233987_m1" "Hs01560931_m1" |
| | "Hs00379134_m1" "Hs00167524_m1" "Hs00826827_g1" "Hs00978280_m1" |
| | "Hs00904817_m1" "Hs00856927_g1" "Hs00899658_m1" "Hs01008033_m1" |
| | "Hs00931763_m1" "Hs00365956_m1" "Hs03302824_pri" "Hs00428732_m1" |
| | "Hs00975850_m1" "Hs01894962_s1" "Hs00703025_s1" "Hs01072228_m1" |
| | "Hs01098278_m1" "Hs00958111_m1" "Hs00944507_g1" "Hs01010736_m1" |
| | "Hs00996236_m1" "Hs01920599_gH" "Hs00387770_m1" "Hs01547054_m1" |
| | "Hs00610058_m1" "Hs01087946_g1" |
| 30-13 | "Hs00856927 _gl" "Hs00915710_m1" "Hs00379134_m1" "Hs00233987_m1" |
| | "Hs01008033_m1" "Hs00167524_m1" "Hs00899658_m1" "Hs00559914_m1" |
| | "Hs00904817_m1" "Hs04942686_m1" "Hs00386692_m1" "Hs00704853_s1" |
| | "Hs00978280_m1" "Hs00415546_m1" "Hs00399035_m1" "Hs00374264_g1" |
| | "Hs01560931_m1" "Hs01546752_g1" "Hs00231709_m1" "Hs00387770_m1" |
| | "Hs00964384_g1" "Hs00698959 _m1" "Hs00196245_m1" "Hs01395177_m1" |
| | "Hs01076090_m1" "Hs00201707_m1" "Hs03302824_pri" "Hs00703025_s1" |
| | "Hs01114274 ml" "Hs01631495_s1" |
| 30-14 | "Hs00380101_m1" "Hs00232157_m1" "Hs00428732_m1" "Hs03302824_pri" |
| | "Hs01031740_m1" "Hs00171042_m1" "Hs01549976_m1" "Hs00260452_m1" |
| | "Hs00745492_s1" "Hs00411188_g1" "Hs00167524_m1" "Hs01560931_m1" |
| | "Hs00356958_m1" "Hs00559914_m1" "Hs00262107_m1" "Hs03464469_s1" |
| | "Hs00801390_s1" "Hs00202752_m1" "Hs00233987_m1" "Hs00196245_m1" |
| | "Hs00960591_m1" "Hs01546752_g1" "Hs01087946_g1" "Hs01547054_m1" |
| | "Hs00260480_m1" "Hs01070154_m1" "Hs00267207_m1" "Hs00174705_m1" |
| | "Hs00747379 _m1" "Hs00387770_m1" |
| 30-15 | "Hs00960591_m1" "Hs00231709_m1" "Hs00251883_m1" "Hs00387770_m1" |
| | "Hs00356958_m1" "Hs00196245_m1" "Hs00174969_m1" "Hs00996236_m1" |
| | "Hs00559914_m1" "Hs00962398_m1" "Hs00856927_g1" "Hs00826827_g1" |
| | "Hs01025572_m1" "Hs00801390_s1" "Hs03043789_g1" "Hs00950371_m1" |
| | "Hs00233987_m1" "Hs00202752_m1" "Hs00260480_m1" "Hs00944507_g1" |
| | "Hs00978280_m1" "Hs01894962_s1" "Hs00204257_m1" "Hs00174029_m1" |
| | "Hs00703025_s1" "Hs01051611_gH" "Hs00958111_m1" "Hs00262107_m1" |
| | "Hs00167524_m1" "Hs01098278_m1" |
| 30-16 | "Hs00944507_g1" "Hs05016463_s1" "Hs03464469_s1" "Hs00167051_m1" |
| | "Hs00900510_m1" "Hs00904817_m1" "Hs04232205_s1" "Hs00356958_m1" |
| | "Hs00957562_m1" "Hs00745492_s1" "Hs00931763_m1" "Hs01043717_m1" |
| | "Hs00167524_m1" "Hs00856927_g1" "Hs00992679_m1" "Hs00380101_m1" |
| | "Hs01549976_m1" "Hs00559914_m1" "Hs01560931_m1" "Hs01098278_m1" |
| | "Hs00703025_s1" "Hs00248075_m1" "Hs01081598_m1" "Hs00428732_m1" |
| | "Hs00968305_m1" "Hs00705626_s1" "Hs00386692_m1" "Hs00174705_m1" |
| | "Hs00251883_m1" "Hs00415546_m1" |
| 30-17 | "Hs00543973_m1" "Hs00159178_m1" "Hs01098278_m1" "Hs00399035_m1" |
| | "Hs01029174_m1" "Hs00705626_s1" "Hs03302824_pri" "Hs01051611_gH" |
| | "Hs05033260_s1" "Hs01560931_m1" "Hs00167524_m1" "Hs01042796_m1" |
| | "Hs05052601_s1" "Hs00944507_g1" "Hs00978280_m1" "Hs00747379 _m1" |
| | "Hs00171042_m1" "Hs00251883_m1" "Hs00960591_m1" "Hs01072228_m1" |
| | "Hs03043789_g1" "Hs00233987_m1" "Hs04942686_m1" "Hs00801390_s1" |
| | "Hs00992679_m1" "Hs01010736_m1" "Hs00232157_m1" "Hs00896999_g1" |
| | "Hs00826827_g1" "Hs00231709_m1" |
| 30-18 | "Hs01920599_gH" "Hs00248075_m1" "Hs00411188_g1" "Hs01560931_m1" |
| | "Hs00900510_m1" "Hs00996236_m1" "Hs00745492_s1" "Hs00202752_m1" |
| | "Hs00233987_m1" "Hs00222415_m1" "Hs01089557_s1" "Hs05052601_s1" |
| | "Hs00960591_m1" "Hs00958111_m1" "Hs01379134_m1" "Hs00380101_m1" |
| | "Hs00856927_g1" "Hs04189864_m1" "Hs01031740_m1" "Hs01042796_m1" |
| | "Hs01098278_m1" "Hs01395177_m1" "Hs00950371_m1" "Hs01691258_g1" |
| | "Hs00899658_m1" "Hs00962398_m1" "Hs00240906_m1" "Hs00399035_m1" |
| | "Hs00428732_m1" "Hs04942686_m1" |
| 30-19 | "Hs00428732_m1" "Hs01560931_m1" "Hs01025572_m1" "Hs00232157_m1" |
| | "Hs01089557_s1" "Hs01043717_m1" "Hs00705626_s1" "Hs00884853_s1" |
| | "Hs00543973_m1" "Hs00992679_m1" "Hs00899658_m1" "Hs00233987_m1" |
| | "Hs00745492_s1" "Hs01031740_m1" "Hs01076090_m1" "Hs00260480_m1" |
| | "Hs00856927_g1" "Hs00189880_m1" "Hs00231709_m1" "Hs01920599_gH" |
| | "Hs00171042_m1" "Hs00415546_m1" "Hs01894962_s1" "Hs04189864_m1" |
| | "Hs00174969_m1" "Hs00610058_m1" "Hs00153408_m1" "Hs01379134_m1" |
| | "Hs00915710_m1" "Hs01395177_m1" |
| 30-20 | "Hs00267207_m1" "Hs00366766_m1" "Hs04942686_m1" "Hs00231709_m1" |
| | "Hs01098278_m1" "Hs05016463_s1" "Hs00155241_m1" "Hs00900510_m1" |
| | "Hs00975850_m1" "Hs04194422_s1" "Hs01114274_m1" "Hs01072228_m1" |
| | "Hs01089557_s1" "Hs00747379_m1" "Hs00944507_g1" "Hs00705626_s1" |
| | "Hs00202752_m1" "Hs00978280_m1" "Hs01920599_gH" "Hs00196245_m1" |
| | "Hs04189864_m1" "Hs01025572_m1" "Hs05052601_s1" "Hs00380101_m1" |
| | "Hs01081598_m1" "Hs00372831_g1" "Hs00167051_m1" "Hs00232157_m1" |
| | "Hs01553775_g1" "Hs00738791_g1" |
| 30-21 | "Hs00174705_m1" "Hs01042796_m1" "Hs00856927_g1" "Hs00372831_g1" |
| | "Hs03302824_pri" "Hs05033260_s1" "Hs04189864_m1" "Hs00992679_m1" |
| | "Hs01560931_m1" "Hs00159178_m1" "Hs00196245_m1" "Hs05052601_s1" |
| | "Hs01031740_m1" "Hs00233987_m1" "Hs00975850_m1" "Hs01098278_m1" |
| | "Hs00248075_m1" "Hs00167524_m1" "Hs00171042_m1" "Hs01920599_gH" |
| | "Hs00380101_m1" "Hs00559914_m1" "Hs04942686_m1" "Hs00202752_m1" |
| | "Hs00937509_m1" "Hs00978280_m1" "Hs00936519_m1" "Hs00222415_m1" |
| | "Hs00365956_m1" "Hs00379134_m1" |
| 30-22 | "Hs00380101_m1" "Hs00233987_m1" "Hs00171042_m1" "Hs00411188_g1" |
| | "Hs00248075_m1" "Hs00559914_m1" "Hs00826827_g1" "Hs01894962_s1" |
| | "Hs01098278_m1" "Hs00232157_m1" "Hs01920599_gH" "Hs00975850_m1" |
| | "Hs00356958_m1" "Hs00543973_m1" "Hs01029174_m1" "Hs00159178_m1" |
| | "Hs05052601_s1" "Hs04194422_s1" "Hs01025572_m1" "Hs00950371_m1" |
| | "Hs00167524_m1" "Hs01072228_m1" "Hs00900510_m1" "Hs00705626_s1" |
| | "Hs01114274_m1" "Hs00904817_m1" "Hs00231709_m1" "Hs00745492_s1" |
| | "Hs00155241_m1" "Hs01081598_m1" |
| 30-23 | "Hs00964384_g1" "Hs00899658_m1" "Hs00374264_g1" "Hs00399035_m1" |
| | "Hs00703025_s1" "Hs00415546_m1" "Hs00232157_m1" "Hs00387770_m1" |
| | "Hs00826827_g1" "Hs01395177_m1" "Hs00202752_m1" "Hs00171042_m1" |
| | "Hs00996236_m1" "Hs00937509_m1" "Hs05016463_s1" "Hs00233987_m1" |
| | "Hs01114274_m1" "Hs01042796_m1" "Hs00248075_m1" "Hs00957562_m1" |
| | "Hs00196245 m1" "Hs01560931_m1" "Hs01089557_s1" "Hs00222415_m1" |
| | "Hs04194422_s1" "Hs01931732_s1" "Hs00884853_s1" "Hs00978280_m1" |
| | "Hs00959010_m1" "Hs00559914_m1" |
| 30-24 | "Hs01042796_m1" "Hs00801390_s1" "Hs00960591_m1" "Hs00992679_m1" |
| | "Hs00978280_m1" "Hs00937509_m1" "Hs01931732_s1" "Hs01560931_m1" |
| | "Hs05016463_s1" "Hs00171157_m1" "Hs00167524_m1" "Hs00260452_m1" |
| | "Hs01089557_s1" "Hs00232157_m1" "Hs00899658_m1" "Hs00904817_m1" |
| | "Hs00202752_m1" "Hs00958111_m1" "Hs00968305_m1" "Hs00248075_m1" |
| | "Hs00607978_s1" "Hs04942686_m1" "Hs01920599_gH" "Hs00365956_m1" |
| | "Hs00944507_g1" "Hs01043717_m1" "Hs00745492_s1" "Hs00704853_s1" |
| | "Hs00610058_m1" "Hs04194422_s1" |
| 30-25 | "Hs00738791_g1" "Hs04232205_s1" "Hs00362096_m1" "Hs00260452_m1" |
| | "Hs01547054_m1" "Hs00196245_m1" "Hs01560931_m1" "Hs00964384_g1" |
| | "Hs00415546_m1" "Hs00202752_m1" "Hs03043789_g1" "Hs00997579_m1" |
| | "Hs00745492_s1" "Hs00174029_m1" "Hs04194422_s1" "Hs00899658_m1" |
| | "Hs00251883_m1" "Hs00915710_m1" "Hs01042796_m1" "Hs04942686_m1" |
| | "Hs00248075_m1" "Hs00222415_m1" "Hs01081598_m1" "Hs01089557_s1" |
| | "Hs00705626_s1" "Hs00356958_m1" "Hs00204257_m1" "Hs00926053_m1" |
| | "Hs05052601_s1" "Hs00372831_g1" |
| 40-1 | "Hs00262107 ml" "Hs01560931_m1" "Hs00159178_m1" "Hs00975850_m1" |
| | "Hs00366766_m1" "Hs00543973_m1" "Hs00231709_m1" "Hs00372831_g1" |
| | "Hs00745492_s1" "Hs01072228_m1" "Hs04194422_s1" "Hs00174029_m1" |
| | "Hs00171157_m1" "Hs01029174_m1" "Hs01089557_s1" "Hs00801390_s1" |
| | "Hs00958111_m1" "Hs01098278_m1" "Hs01920599_gH" "Hs00233987_m1" |
| | "Hs00374264_gl" "Hs04189864_m1" "Hs00155241_m1" "Hs00610058_m1" |
| | "Hs03464469_s1" "Hs00386692_m1" "Hs00964384_g1" "Hs01025572_m1" |
| | "Hs01546752_g1" "Hs01395177_m1" "Hs00248075_m1" "Hs00937509_m1" |
| | "Hs00704853_s1" "Hs03028557_s1" "Hs00747379_m1" "Hs00904817_m1" |
| | "Hs00362096_m1" "Hs01087946_g1" "Hs01031740_m1" "Hs00978280_m1" |
| 40-2 | "Hs00231709_m1" "Hs01098278_m1" "Hs00174969_m1" "Hs01029174_m1" |
| | "Hs05036222_s1" "Hs00262107_m1" "Hs00856927_g1" "Hs05052601_s1" |
| | "Hs00233987_m1" "Hs00559914_m1" "Hs01931732_s1" "Hs01560931_m1" |
| | "Hs00167524_m1" "Hs00543973_m1" "Hs01010736_m1" "Hs00174029_m1" |
| | "Hs00260480_m1" "Hs00996236_m1" "Hs00745492_s1" "Hs00204257_m1" |
| | "Hs00374264_g1" "Hs04942686_m1" "Hs00411188_g1" "Hs00380101_m1" |
| | "Hs01051611_gH" "Hs01089557 _s1" "Hs00901350_g1" "Hs01081598_m1" |
| | "Hs00738791_g1" "Hs01025572_m1" "Hs00248075_m1" "Hs01894962_s1" |
| | "Hs00957562_m1" "Hs00189880_m1" "Hs00937509_m1" "Hs00362096_m1" |
| | "Hs00962398_m1" "Hs00171042_m1" "Hs01076090_m1" "Hs00801390_s1" |
| 40-3 | "Hs00978280_m1" "Hs00900510_m1" "Hs00411188_g1" "Hs00174969_m1" |
| | "Hs00171157_m1" "Hs01546752_g1" "Hs04194422_s1" "Hs00826827_g1" |
| | "Hs01010736 m1" "Hs01043717_m1" "Hs00931763_m1" "Hs01089557_s1" |
| | "Hs00703025_s1" "Hs00559914_m1" "Hs03302824_pri" "Hs01087946_g1" |
| | "Hs00950371_m1" "Hs01051445_g1" "Hs00202752_m1" "Hs00380101_m1" |
| | "Hs01553775_g1" "Hs00232157_m1" "Hs00698959_m1" "Hs01098278_m1" |
| | "Hs03464469_s1" "Hs00884853_s1" "Hs00926053_m1" "Hs00936519_m1" |
| | "Hs00745492_s1" "Hs01560931_m1" "Hs00362096_m1" "Hs00204257_m1" |
| | "Hs00240906_m1" "Hs00366766_m1" "Hs00260480_m1" "Hs01549976_m1" |
| | "Hs00997579_m1" "Hs00738791_g1" "Hs01114274 m1" "Hs03043789_g1" |
| 40-4 | "Hs00899658 ml" "Hs00543973_m1" "Hs00260452_m1" "Hs00233987_m1" |
| | "Hs03302824_pri" "Hs04189864_m1" "Hs01089557_s1" "Hs05036222_s1" |
| | "Hs00428732_m1" "Hs00374264_g1" "Hs00958111_m1" "Hs00167524_m1" |
| | "Hs00856927_g1" "Hs00964384_g1" "Hs00900510_m1" "Hs00747379 _m1" |
| | "Hs01087946_g1" "Hs05033260_s1" "Hs00607978_s1" "Hs04942686_m1" |
| | "Hs00937509 m1" "Hs00380101_m1" "Hs00386692_m1" "Hs00155241_m1" |
| | "Hs01114274_m1" "Hs03028557_s1" "Hs00240906_m1" "Hs01546752_g1" |
| | "Hs00975850_m1" "Hs00610058_m1" "Hs01631495_s1" "Hs00174029_m1" |
| | "Hs00411188_g1" "Hs00559914_m1" "Hs03043789_g1" "Hs00698959 _m1" |
| | "Hs00703025_s1" "Hs00745492_s1" "Hs00387770_m1" "Hs01008033_m1" |
| 40-5 | "Hs00607978_s1" "Hs01031740_m1" "Hs00174969_m1" "Hs01114274_m1" |
| | "Hs04942686_m1" "Hs00931763_m1" "Hs00996236_m1" "Hs01395177_m1" |
| | "Hs01098278_m1" "Hs00387770_m1" "Hs04194422_s1" "Hs00248075_m1" |
| | "Hs01546752_g1" "Hs00705626_s1" "Hs01920599_gH" "Hs00801390_s1" |
| | "Hs05052601_s1" "Hs00960591_m1" "Hs01076090_m1" "Hs00232157_m1" |
| | "Hs00262107_m1" "Hs00944507_g1" "Hs00174705_m1" "Hs01089557_s1" |
| | "Hs01029174_m1" "Hs01051445_g1" "Hs00362096_m1" "Hs00399035_m1" |
| | "Hs00745492_s1" "Hs00978280_m1" "Hs01043717_m1" "Hs03028557_s1" |
| | "Hs00233987_m1" "Hs00704853_s1" "Hs01008033_m1" "Hs00543973_m1" |
| | "Hs00251883_m1" "Hs01691258_g1" "Hs00155241_m1" "Hs01042796_m1" |
| 40-6 | "Hs01931732_s1" "Hs00174705_m1" "Hs00248075_m1" "Hs00174969_m1" |
| | "Hs00950371_m1" "Hs00374264_g1" "Hs00232157_m1" "Hs00747379 _m1" |
| | "Hs00884853_s1" "Hs00411188_g1" "Hs01089557_s1" "Hs00196245_m1" |
| | "Hs01029174_m1" "Hs01560931_m1" "Hs00379134_m1" "Hs04942686_m1" |
| | "Hs00738791_g1" "Hs00960591_m1" "Hs00171042_m1" "Hs05016463_s1" |
| | "Hs01025572 m1" "Hs00260480_m1" "Hs00428732_m1" "Hs00705626_s1" |
| | "Hs01114274_m1" "Hs00153408_m1" "Hs00944507_g1" "Hs00171157_m1" |
| | "Hs00962398_m1" "Hs00975850_m1" "Hs00997579_m1" "Hs00931763_m1" |
| | "Hs01379134_m1" "Hs04194422_s1" "Hs00703025_s1" "Hs01553775_g1" |
| | "Hs00365956_m1" "Hs01098278_m1" "Hs00559914_m1" "Hs00992679_m1" |
| 40-7 | "Hs00387770_m1" "Hs01031740_m1" "Hs00356958_m1" "Hs00959010_m1" |
| | "Hs01395177_m1" "Hs00705626_s1" "Hs01010736_m1" "Hs00703025_s1" |
| | "Hs00801390_s1" "Hs01894962_s1" "Hs00233987_m1" "Hs00174029_m1" |
| | "Hs00196245 m1" "Hs01051445_g1" "Hs00153408_m1" "Hs01081598_m1" |
| | "Hs00411188_g1" "Hs01089557_s1" "Hs01920599_gH" "Hs00901350_g1" |
| | "Hs00950371_m1" "Hs00997579_m1" "Hs00415546_m1" "Hs00884853_s1" |
| | "Hs00155241_m1" "Hs00915710_m1" "Hs03302824_pri" "Hs01025572_m1" |
| | "Hs00698959_m1" "Hs00167524_m1" "Hs00936519_m1" "Hs00992679_m1" |
| | "Hs00704853_s1" "Hs00960591_m1" "Hs00968305_m1" "Hs00380101_m1" |
| | "Hs00745492_s1" "Hs00201707_m1" "Hs00996236_m1" "Hs00366766_m1" |
| 40-8 | "Hs00155241_m1" "Hs01025572_m1" "Hs01087946_g1" "Hs00204257_m1" |
| | "Hs01395177_m1" "Hs05052601_s1" "Hs00962398_m1" "Hs03028557_s1" |
| | "Hs05033260_s1" "Hs00610058_m1" "Hs00174029_m1" "Hs05036222_s1" |
| | "Hs04189864_m1" "Hs00543973_m1" "Hs01081598_m1" "Hs01894962_s1" |
| | "Hs00738791_g1" "Hs00171042_m1" "Hs00957562_m1" "Hs01560931_m1" |
| | "Hs00231709 m1" "Hs01098278_m1" "Hs00703025_s1" "Hs01089557_s1" |
| | "Hs00374264_g1" "Hs01010736_m1" "Hs00901350_g1" "Hs00174969_m1" |
| | "Hs00260480_m1" "Hs00996236_m1" "Hs05016463_s1" "Hs00415546_m1" |
| | "Hs00362096_m1" "Hs00704853_s1" "Hs00975850_m1" "Hs00174705_m1" |
| | "Hs00251883_m1" "Hs00196245_m1" "Hs00222415_m1" "Hs01931732_s1" |
| 40-9 | "Hs00267207 m1" "Hs00904817_m1" "Hs01087946_g1" "Hs00233987_m1" |
| | "Hs04194422_s1" "Hs00196245_m1" "Hs03302824_pri" "Hs01089557_s1" |
| | "Hs00936519_m1" "Hs01560931_m1" "Hs00559914_m1" "Hs00171157_m1" |
| | "Hs00900510_m1" "Hs00174705_m1" "Hs00856927_g1" "Hs00950371_m1" |
| | "Hs01043717_m1" "Hs00960591_m1" "Hs01546752_g1" "Hs00957562_m1" |
| | "Hs00372831_g1" "Hs01029174_m1" "Hs00937509 _m1" "Hs00411188_g1" |
| | "Hs05016463_s1" "Hs00705626_s1" "Hs00232157_m1" "Hs00174029_m1" |
| | "Hs00978280_m1" "Hs05052601_s1" "Hs05033260_s1" "Hs01920599_gH" |
| | "Hs00231709_m1" "Hs01098278_m1" "Hs01081598_m1" "Hs00997579_m1" |
| | "Hs01051445_g1" "Hs04232205_s1" "Hs00884853_s1" "Hs00738791_g1" |
| 40-10 | "Hs01076090_m1" "Hs00387770_m1" "Hs01031740_m1" "Hs00196245_m1" |
| | "Hs03464469_s1" "Hs04232205_s1" "Hs00884853_s1" "Hs00174969_m1" |
| | "Hs00607978_s1" "Hs04942686_m1" "Hs00931763_m1" "Hs01098278_m1" |
| | "Hs00996236_m1" "Hs00960591_m1" "Hs01920599_gH" "Hs01546752_g1" |
| | "Hs00997579_m1" "Hs01087946_g1" "Hs01560931_m1" "Hs00950371_m1" |
| | "Hs01549976_m1" "Hs00747379_m1" "Hs01081598_m1" "Hs01691258_g1" |
| | "Hs00231709_m1" "Hs00399035_m1" "Hs00428732_m1" "Hs00543973_m1" |
| | "Hs00240906_m1" "Hs00372831_g1" "Hs00703025 _s1" "Hs00171042_m1" |
| | "Hs01043717_m1" "Hs01025572_m1" "Hs00222415_m1" "Hs00937509_m1" |
| | "Hs00267207_m1" "Hs00915710_m1" "Hs00366766_m1" "Hs00610058_m1" |
| 40-11 | "Hs00610058_m1" "Hs00745492_s1" "Hs00901350_g1" "Hs01051611_gH" |
| | "Hs01087946_g1" "Hs05052601_s1" "Hs03028557_s1" "Hs00248075_m1" |
| | "Hs01089557_s1" "Hs00738791_g1" "Hs00374264_g1" "Hs00543973_m1" |
| | "Hs04189864_m1" "Hs00698959_m1" "Hs05036222_s1" "Hs00962398_m1" |
| | "Hs00362096_m1" "Hs03043789_g1" "Hs03302824_pri" "Hs00380101_m1" |
| | "Hs00232157_m1" "Hs01553775_g1" "Hs01547054_m1" "Hs00174705_m1" |
| | "Hs00936519_m1" "Hs00386692_m1" "Hs00926053_m1" "Hs00996236_m1" |
| | "Hs00960591_m1" "Hs01098278_m1" "Hs01560931_m1" "Hs00202752_m1" |
| | "Hs04942686_m1" "Hs00428732_m1" "Hs01072228_m1" "Hs00884853_s1" |
| | "Hs00931763_m1" "Hs00964384_g1" "Hs00826827_g1" "Hs00155241_ml" |
| 40-12 | "Hs00174705_m1" "Hs00698959_m1" "Hs01546752_g1" "Hs00262107_m1" |
| | "Hs03464469_s1" "Hs00996236_m1" "Hs00960591_m1" "Hs00387770_m1" |
| | "Hs00204257_m1" "Hs00801390_s1" "Hs00610058_m1" "Hs01631495_s1" |
| | "Hs01081598_m1" "Hs00240906_m1" "Hs01553775_g1" "Hs00362096_m1" |
| | "Hs00899658_m1" "Hs00248075_m1" "Hs01894962_s1" "Hs01089557_s1" |
| | "Hs00747379_m1" "Hs01560931_m1" "Hs00267207_m1" "Hs01010736_m1" |
| | "Hs00901350_g1" "Hs03302824_pri" "Hs01547054 _m1" "Hs00386692_m1" |
| | "Hs01098278_m1" "Hs00399035_m1" "Hs00189880_m1" "Hs00232157_m1" |
| | "Hs00356958_m1" "Hs00167524_m1" "Hs00380101_m1" "Hs04942686_m1" |
| | "Hs00174969_m1" "Hs00968305_m1" "Hs00745492_s1" "Hs00366766_m1" |
| 40-13 | "Hs01546752_g1" "Hs01920599_gH" "Hs00801390_s1" "Hs01560931_m1" |
| | "Hs04194422_s1" "Hs01081598_m1" "Hs00251883_m1" "Hs00559914_m1" |
| | "Hs00262107_m1" "Hs00374264_g1" "Hs00543973_m1" "Hs00171157_m1" |
| | "Hs00937509_m1" "Hs01029174_m1" "Hs00174969_m1" "Hs00232157_m1" |
| | "Hs00705626_s1" "Hs05016463_s1" "Hs01553775_g1" "Hs00704853_s1" |
| | "Hs01691258_g1" "Hs00167524_m1" "Hs00962398_m1" "Hs00978280_m1" |
| | "Hs00248075_m1" "Hs00386692_m1" "Hs00959010_m1" "Hs01098278_m1" |
| | "Hs00884853_s1" "Hs00174705_m1" "Hs00996236_m1" "Hs00379134_m1" |
| | "Hs04942686_m1" "Hs00202752_m1" "Hs00189880_m1" "Hs00365956_m1" |
| | "Hs03302824_pri" "Hs00926053_m1" "Hs01395177_m1" "Hs04189864_m1" |
| 40-14 | "Hs00747379_m1" "Hs00362096_m1" "Hs00386692_m1" "Hs01089557_s1" |
| | "Hs01029174_m1" "Hs00960591_m1" "Hs00248075_m1" "Hs01547054_m1" |
| | "Hs01631495_s1" "Hs00189880_m1" "Hs00167524_m1" "Hs01098278_m1" |
| | "Hs00996236_m1" "Hs00240906_m1" "Hs01087946_g1" "Hs00884853_s1" |
| | "Hs00899658_m1" "Hs00380101_m1" "Hs04942686_m1" "Hs00936519_m1" |
| | "Hs00856927_g1" "Hs01043717_m1" "Hs00915710_m1" "Hs00231709_m1" |
| | "Hs00964384_g1" "Hs00387770_m1" "Hs00155241_m1" "Hs01081598_m1" |
| | "Hs00926053_m1" "Hs00366766_m1" "Hs00171042_m1" "Hs01031740_m1" |
| | "Hs00959010_m1" "Hs01546752_g1" "Hs00233987_m1" "Hs01042796_m1" |
| | "Hs00896999_g1" "Hs00374264_g1" "Hs04232205_s1" "Hs00201707_m1" |
| 40-15 | "Hs00703025_s1" "Hs01087946_g1" "Hs05016463_s1" "Hs00167524_m1" |
| | "Hs00543973_m1" "Hs01010736_m1" "Hs05052601_s1" "Hs00957562_m1" |
| | "Hs00856927_g1" "Hs04232205_s1" "Hs01029174_m1" "Hs00251883_m1" |
| | "Hs01931732_s1" "Hs00262107_m1" "Hs00374264_g1" "Hs01560931_m1" |
| | "Hs00174969_m1" "Hs00937509_m1" "Hs00222415_m1" "Hs00801390_s1" |
| | "Hs00959010_m1" "Hs00231709_m1" "Hs00747379_m1" "Hs00411188_g1" |
| | "Hs01051445_g1" "Hs00174029_m1" "Hs01553775_g1" "Hs01072228_m1" |
| | "Hs01089557_s1" "Hs00415546_m1" "Hs00705626_s1" "Hs00387770_m1" |
| | "Hs00745492_s1" "Hs00153408_m1" "Hs00196245 _m1" "Hs00884853_s1" |
| | "Hs04189864_m1" "Hs00936519_m1" "Hs00958111_m1" "Hs00607978_s1" |
| 40-16 | "Hs01920599_gH" "Hs04189864_m1" "Hs01114274_m1" "Hs01395177_m1" |
| | "Hs00374264_g1" "Hs00174029_m1" "Hs00958111_m1" "Hs03028557_s1" |
| | "Hs00856927_g1" "Hs00153408_m1" "Hs01089557_s1" "Hs00607978_s1" |
| | "Hs00201707_m1" "Hs00155241_m1" "Hs00233987_m1" "Hs00745492_s1" |
| | "Hs00610058_ml" "Hs00964384_g1" "Hs04942686_m1" "Hs00996236_m1" |
| | "Hs00174969_m1" "Hs00944507_g1" "Hs00248075_m1" "Hs00362096_m1" |
| | "Hs00174705_m1" "Hs01043717_m1" "Hs00260452_m1" "Hs00167524_m1" |
| | "Hs00380101_m1" "Hs00559914_m1" "Hs00159178_m1" "Hs04194422_s1" |
| | "Hs00399035_m1" "Hs01931732_s1" "Hs00703025_s1" "Hs00738791_g1" |
| | "Hs00826827_g1" "Hs00997579_m1" "Hs00204257_m1" "Hs01070154_m1" |
| 40-17 | "Hs00978280_m1" "Hs01553775_g1" "Hs01029174_m1" "Hs00996236_m1" |
| | "Hs00386692_m1" "Hs00738791_g1" "Hs01089557_s1" "Hs00365956_m1" |
| | "Hs00607978_s1" "Hs00248075_m1" "Hs01098278_m1" "Hs00962398_m1" |
| | "Hs00196245_m1" "Hs00232157_m1" "Hs00167524_m1" "Hs04942686_m1" |
| | "Hs00201707_m1" "Hs00915710_m1" "Hs01549976_m1" "Hs00901350_g1" |
| | "Hs04194422_s1" "Hs00997579_m1" "Hs01031740_m1" "Hs00904817_m1" |
| | "Hs01631495_s1" "Hs00153408_m1" "Hs00975850_m1" "Hs00428732_m1" |
| | "Hs00366766_m1" "Hs04232205_s1" "Hs01547054_m1" "Hs00900510_m1" |
| | "Hs01560931_m1" "Hs00747379_m1" "Hs00356958_m1" "Hs00899658_m1" |
| | "Hs00415546_m1" "Hs00362096_m1" "Hs00937509_m1" "Hs00958111_m1" |
| 40-18 | "Hs00201707_m1" "Hs04942686_m1" "Hs04194422_s1" "Hs00899658_m1" |
| | "Hs00260452_m1" "Hs01043717_m1" "Hs00262107_m1" "Hs01070154_m1" |
| | "Hs01029174_m1" "Hs00856927_g1" "Hs01098278_m1" "Hs00996236_m1" |
| | "Hs00944507_g1" "Hs00372831_g1" "Hs00356958_m1" "Hs01051611_gH" |
| | "Hs00380101_m1" "Hs01076090_m1" "Hs00703025_s1" "Hs00251883_m1" |
| | "Hs01560931_m1" "Hs00171157_m1" "Hs00386692_m1" "Hs05016463_s1" |
| | "Hs00167524_m1" "Hs01089557_s1" "Hs00171042_m1" "Hs00745492_s1" |
| | "Hs00610058_m1" "Hs00884853_s1" "Hs00958111_m1" "Hs04189864_m1" |
| | "Hs00704853_s1" "Hs00543973_m1" "Hs00374264_g1" "Hs01087946_g1" |
| | "Hs00415546_m1" "Hs05036222_s1" "Hs00904817_m1" "Hs01072228_m1" |
| 40-19 | "Hs01072228_m1" "Hs00915710_m1" "Hs00174705_m1" "Hs00356958_m1" |
| | "Hs00174029_m1" "Hs00958111_m1" "Hs00248075_m1" "Hs01553775_g1" |
| | "Hs01098278_m1" "Hs00957562_m1" "Hs00937509_m1" "Hs00960591_m1" |
| | "Hs00747379_m1" "Hs00387770_m1" "Hs01547054_m1" "Hs00698959_m1" |
| | "Hs00399035_m1" "Hs00607978_s1" "Hs04942686_m1" "Hs00240906_m1" |
| | "Hs00962398_m1" "Hs00232157_m1" "Hs00267207_m1" "Hs00996236_m1" |
| | "Hs00704853_s1" "Hs00365956_m1" "Hs00196245_m1" "Hs00959010_m1" |
| | "Hs00884853_s1" "Hs01691258_g1" "Hs00950371_m1" "Hs00260480_m1" |
| | "Hs00174969_m1" "Hs01560931_m1" "Hs00964384_g1" "Hs00738791_g1" |
| | "Hs00543973_m1" "Hs00171042_m1" "Hs04189864_m1" "Hs00415546_m1" |
| 40-20 | "Hs00559914_m1" "Hs00856927_g1" "Hs00232157_m1" "Hs01029174_m1" |
| | "Hs00978280_m1" "Hs01051445_g1" "Hs01031740_m1" "Hs00899658_m1" |
| | "Hs00174705_m1" "Hs00153408_m1" "Hs00362096_m1" "Hs00944507_g1" |
| | "Hs00260452_m1" "Hs00937509_m1" "Hs00826827_g1" "Hs00248075_m1" |
| | "Hs01043717_m1" "Hs01070154_m1" "Hs01076090_m1" "Hs01560931_m1" |
| | "Hs01920599_gH" "Hs00543973_m1" "Hs00380101_m1" "Hs00997579_m1" |
| | "Hs03043789_g1" "Hs00901350_g1" "Hs03302824_pri" "Hs00399035_m1" |
| | "Hs00747379_m1" "Hs01114274_m1" "Hs04232205_s1" "Hs05016463_s1" |
| | "Hs00387770_m1" "Hs00366766_m1" "Hs04194422_s1" "Hs01008033_m1" |
| | "Hs01395177_m1" "Hs00904817_m1" "Hs03464469_s1" "Hs01894962_s1" |
| 40-21 | "Hs00826827_g1" "Hs00899658_m1" "Hs01076090_m1" "Hs00411188_g1" |
| | "Hs05036222_s1" "Hs00171042_m1" "Hs00926053_m1" "Hs00233987_m1" |
| | "Hs00174029_m1" "Hs00902334_m1" "Hs00958111_m1" "Hs00153408_m1" |
| | "Hs01081598_m1" "Hs01051445_g1" "Hs04189864_m1" "Hs00962398_m1" |
| | "Hs00155241_m1" "Hs00380101_m1" "Hs01560931_m1" "Hs00167524_m1" |
| | "Hs00171157_m1" "Hs00260480_m1" "Hs00174969_m1" "Hs01114274_m1" |
| | "Hs00705626_s1" "Hs01920599_gH" "Hs00428732_m1" "Hs01031740_m1" |
| | "Hs05016463_s1" "Hs01549976_m1" "Hs00931763_m1" "Hs00703025_ s1" |
| | "Hs00960591_m1" "Hs05052601_s1" "Hs00937509_m1" "Hs01098278_m1" |
| | "Hs00968305_m1" "Hs01087946_g1" "Hs00959010_m1" "Hs01089557_s1" |
| 40-22 | "Hs00904817_m1" "Hs00260480_m1" "Hs00856927_g1" "Hs01894962_s1" |
| | "Hs00171042_m1" "Hs00171157_m1" "Hs01098278_m1" "Hs00202752_m1" |
| | "Hs01076090_m1" "Hs00964384_g1" "Hs00155241_m1" "Hs00950371_m1" |
| | "Hs01042796_m1" "Hs00826827_g1" "Hs00958111_m1" "Hs00899658_m1" |
| | "Hs00233987_m1" "Hs00926053_m1" "Hs01029174_m1" "Hs00174029_m1" |
| | "Hs01081598_m1" "Hs00159178_m1" "Hs00975850_m1" "Hs00232157_m1" |
| | "Hs00251883_m1" "Hs00372831_g1" "Hs00957562_m1" "Hs00610058_m1" |
| | "Hs01043717_m1" "Hs05033260_s1" "Hs01920599_gH" "Hs00543973_m1" |
| | "Hs01560931_m1" "Hs00901350_g1" "Hs00705626_s1" "Hs05016463_s1" |
| | "Hs00260452_m1" "Hs01553775_g1" "Hs00153408_m1" "Hs00962398_m1" |
| 40-23 | "Hs00801390_s1" "Hs00174969_m1" "Hs00959010_m1" "Hs00968305_m1" |
| | "Hs01547054_m1" "Hs01072228_m1" "Hs00262107_m1" "Hs01114274_m1" |
| | "Hs00937509_m1" "Hs01051611_gH" "Hs01920599_gH" "Hs01031740_m1" |
| | "Hs00399035_m1" "Hs00962398_m1" "Hs00233987_m1" "Hs00167524_m1" |
| | "Hs00826827_g1" "Hs01010736_m1" "Hs03302824_pri" "Hs01549976_m1" |
| | "Hs00901350_g1" "Hs00975850_m1" "Hs00904817_m1" "Hs00380101_m1" |
| | "Hs00387770_m1" "Hs04232205_s1" "Hs00240906_m1" "Hs00997579_m1" |
| | "Hs01560931_m1" "Hs01631495_s1" "Hs05052601_s1" "Hs00738791_g1" |
| | "Hs00171157_m1" "Hs00232157_m1" "Hs00978280_m1" "Hs00372831_g1" |
| | "Hs00704853_s1" "Hs00745492_s1" "Hs00936519_m1" "Hs00204257_m1" |
| 40-24 | "Hs01549976_m1" "Hs00260480_m1" "Hs01031740_m1" "Hs05052601_s1" |
| | "Hs05016463_s1" "Hs00196245_m1" "Hs01025572_m1" "Hs01042796_m1" |
| | "Hs00944507_g1" "Hs00171157_m1" "Hs00167524_m1" "Hs00962398_m1" |
| | "Hs00428732_m1" "Hs00543973_m1" "Hs01560931_m1" "Hs01087946_g1" |
| | "Hs00960591_m1" "Hs04189864_m1" "Hs00826827_g1" "Hs00380101_m1" |
| | "Hs01691258_g1" "Hs00386692_m1" "Hs00374264_g1" "Hs00745492_s1" |
| | "Hs00884853_s1" "Hs00411188_g1" "Hs00936519_m1" "Hs00240906_m1" |
| | "Hs00232157_m1" "Hs01098278_m1" "Hs03302824_pri" "Hs00248075_m1" |
| | "Hs00801390_s1" "Hs01553775_g1" "Hs03464469_s1" "Hs00738791_g1" |
| | "Hs01631495_s1" "Hs00703025_s1" "Hs00896999_g1" "Hs01010736_m1" |
| 40-25 | "Hs04189864_m1" "Hs00386692_m1" "Hs00926053_m1" "Hs00260452_m1" |
| | "Hs00610058_m1" "Hs00958111_m1" "Hs00704853_s1" "Hs00380101_m1" |
| | "Hs00543973_m1" "Hs00167524_m1" "Hs00747379_m1" "Hs00428732_m1" |
| | "Hs00559914_m1" "Hs00738791_g1" "Hs00260480 _m1" "Hs00703025_s1" |
| | "Hs00975850_m1" "Hs01025572_m1" "Hs01098278_m1" "Hs01560931_m1" |
| | "Hs00745492_s1" "Hs00233987_m1" "Hs00232157_m1" "Hs05016463_s1" |
| | "Hs00607978_s1" "Hs00171042_m1" "Hs00884853_s1" "Hs01691258_g1" |
| | "Hs00196245_m1" "Hs01081598_m1" "Hs00251883_m1" "Hs00904817_m1" |
| | "Hs01114274_m1" "Hs00222415_m1" "Hs01051445_g1" "Hs01042796_m1" |
| | "Hs00356958_m1" "Hs00944507_g1" "Hs00379134_m1" "Hs00705626_s1" |
| *See* Table 11 for gene name associated with each probe ID. | |

## Claims

1. A method for predicting risk of recurrence, metastasis, or both, in a patient with a cutaneous squamous cell carcinoma (cSCC) tumor, the method comprising:
(a) isolating mRNA from a cSCC tumor sample that has been obtained from the patient;
(b) determining the expression level of ALOX12, BBC, BHLHB9, GTPBP2, HDDC3, ID2, LCE2B, LOC100287896, MMP10, MSANTD4, NFASC, NFIC, PDPN, RCHY1, RPP38, RUNX3, TAF6L, TFAP2B, ZNF48, and ZNF496;
(c) providing an indication as to whether the cSCC tumor has a low risk to a high risk of local recurrence, distant metastasis, or both, based on the expression levels of ALOX 12, BBC, BHLHB9, GTPBP2, HDDC3, ID2, LCE2B, LOC100287896, MMP10, MSANTD4, NFASC, NFIC, PDPN, RCHY1, RPP38, RUNX3, TAF6L, TFAP2B, ZNF48, and ZNF496.

2. The method of claim 1, further comprising comparing the expression levels of ALOX12, BBC, BHLHB9, GTPBP2, HDDC3, ID2, LCE2B, LOC100287896, MMP10, MSANTD4, NFASC, NFIC, PDPN, RCHY1, RPP38, RUNX3, TAF6L, TFAP2B, ZNF48, and ZNF49 from the cSCC tumor sample to the expression levels of ALOX12, BBC, BHLHB9, GTPBP2, HDDC3, ID2, LCE2B, LOC100287896, MMP10, MSANTD4, NFASC, NFIC, PDPN, RCHY1, RPP38, RUNX3, TAF6L, TFAP2B, ZNF48, and ZNF49 from a predictive training set to generate a probability score of the risk of local recurrence, distant metastasis, or both.

3. The method of claim 1 or 2, wherein the expression level of each gene in the gene set is determined by reverse transcribing the isolated mRNA into cDNA and measuring a level of fluorescence for each gene in the gene set by a nucleic acid sequence detection system following Real-Time Polymerase Chain Reaction (RT-PCR).

4. The method of any one of claims 1 to 3, wherein the cSCC tumor sample is obtained from a formalin-fixed, paraffin embedded sample.

5. The method of any one of claims 2 to 4, wherein the probability score of local recurrence, distant metastasis, or both is between 0 and 1, wherein a value of 1 indicates a higher probability of local recurrence, distant metastasis, or both than a value of 0, and wherein the probability score from 0 to 1 reflects the correlation of the expression level of the cSCC tumor sample with the training set used to predict risk outcome.

6. The method of any one of claims 2 to 5, wherein the probability score is a bimodal, two-class analysis, wherein a patient having a value of between 0 and 0.499 is designated as class 1 (low risk) and a patient having a value of between 0.500 and 1.00 is designated as class 2 (high risk), and wherein the probability score from 0 to 1 reflects the correlation of the expression level of the cSCC tumor sample with the training set used to predict risk outcome.

7. The method of any one of claims 2 to 6, wherein the probability score is a tri-modal, three-class analysis, wherein patients are designated as class A (low risk), class B (intermediate risk), or class C (high risk).

8. The method of any one of claims 2 to 7, further comprising identifying the cSCC tumor has a high risk of local recurrence, distant metastasis, or both based on the probability score, and administering to the patient an aggressive tumor treatment.

9. The method of any one of claims 1 to 8, wherein the expression level of:
ALOX12 is decreased, BBC is increased, BHLHB9 is decreased, GTPBP2 is decreased, HDDC3 is increased, ID2 is decreased, LCE2B is decreased, LOC100287896 is increased, MMP10 is decreased, MSANTD4 is decreased, NFASC is decreased, NFIC is decreased, PDPN is increased, RCHY1 is increased, RPP38 is decreased, RUNX3 is increased, TAF6L is increased, TFAP2B is decreased, ZNF48 is increased, and ZNF496 is increased when comparing a recurrent tumor to a non-recurrent sample.

## Patentansprüche

1. Verfahren zur Vorhersage des Risikos eines Rezidivs, einer Metastasierung oder beider bei einem Patienten mit einem kutanen Plattenepithelkarzinom(cSCC)-Tumor, wobei das Verfahren umfasst:
(a) Isolieren von mRNA aus einer cSCC-Tumorprobe, die aus dem Patienten erhalten wurde;
(b) Bestimmen des Expressionsniveaus von ALOX12, BBC, BHLHB9, GTPBP2, HDDC3, ID2, LCE2B, LOC100287896, MMP10, MSANTD4, NFASC, NFIC, PDPN, RCHY1, RPP38, RUNX3, TAF6L, TFAP2B, ZNF48 und ZNF496;
(c) Bereitstellen einer Angabe dahingehend, ob der cSCC-Tumor ein niedriges Risiko bis ein hohes Risiko eines lokalen Rezidivs, einer Fernmetastasierung oder beider aufweist, basierend auf den Expressionsniveaus von ALOX12, BBC, BHLHB9, GTPBP2, HDDC3, ID2, LCE2B, LOC100287896, MMP10, MSANTD4, NFASC, NFIC, PDPN, RCHY1, RPP38, RUNX3, TAF6L, TFAP2B, ZNF48 und ZNF496.

2. Verfahren nach Anspruch 1, ferner umfassend den Vergleich der Expressionsniveaus von ALOX12, BBC, BHLHB9, GTPBP2, HDDC3, ID2, LCE2B, LOC100287896, MMP10, MSANTD4, NFASC, NFIC, PDPN, RCHY1, RPP38, RUNX3, TAF6L, TFAP2B, ZNF48 und ZNF49 aus der cSCC-Tumorprobe mit den Expressionsniveaus von ALOX12, BBC, BHLHB9, GTPBP2, HDDC3, ID2, LCE2B, LOC100287896, MMP10, MSANTD4, NFASC, NFIC, PDPN, RCHY1, RPP38, RUNX3, TAF6L, TFAP2B, ZNF48 und ZNF49 aus einem prädiktiven Trainingssatz, um einen Wahrscheinlichkeitsscore des Risikos eines lokalen Rezidivs, einer Fernmetastasierung oder beider zu erzeugen.

3. Verfahren nach Anspruch 1 oder 2, wobei das Expressionsniveau jedes Gens in dem Gensatz bestimmt wird, indem die isolierte mRNA in cDNA revers transkribiert und ein Fluoreszenzniveau für jedes Gen in dem Gensatz durch ein Nukleinsäuresequenz-Detektionssystem nach Echtzeit-Polymerase-Kettenreaktion (RT-PCR) gemessen wird.

4. Verfahren nach einem der Ansprüche 1 bis 3, wobei die cSCC-Tumorprobe aus einer formalinfixierten, in Paraffin eingebetteten Probe erhalten wird.

5. Verfahren nach einem der Ansprüche 2 bis 4, wobei der Wahrscheinlichkeitsscore eines lokalen Rezidivs, einer Fernmetastasierung oder beider zwischen 0 und 1 liegt, wobei ein Wert von 1 eine höhere Wahrscheinlichkeit eines lokalen Rezidivs, einer Fernmetastasierung oder beider angibt als ein Wert von 0 und wobei der Wahrscheinlichkeitsscore von 0 bis 1 die Korrelation des Expressionsniveaus der cSCC-Tumorprobe mit dem Trainingssatz, der zur Vorhersage des Risikoergebnisses verwendet wird, widerspiegelt.

6. Verfahren nach einem der Ansprüche 2 bis 5, wobei der Wahrscheinlichkeitsscore eine bimodale Zwei-Klassen-Analyse ist, wobei ein Patient mit einem Wert zwischen 0 und 0,499 als Klasse 1 (niedriges Risiko) designiert wird und ein Patient mit einem Wert zwischen 0,500 und 1,00 als Klasse 2 (hohes Risiko) designiert wird und wobei der Wahrscheinlichkeitsscore von 0 bis 1 die Korrelation des Expressionsniveaus der cSCC-Tumorprobe mit dem Trainingssatz, der zur Vorhersage des Risikoergebnisses verwendet wird, widerspiegelt.

7. Verfahren nach einem der Ansprüche 2 bis 6, wobei der Wahrscheinlichkeitsscore eine trimodale Drei-Klassen-Analyse ist, wobei Patienten als Klasse A (niedriges Risiko), Klasse B (intermediäres Risiko) oder Klasse C (hohes Risiko) designiert werden.

8. Verfahren nach einem der Ansprüche 2 bis 7, ferner umfassend das Identifizieren, dass der cSCC-Tumors ein hohes Risiko eines lokalen Rezidivs, einer Fernmetastasierung oder beider, basierend auf dem Wahrscheinlichkeitsscore, aufweist und das Verabreichen einer aggressiven Tumorbehandlung an den Patienten.

9. Verfahren nach einem der Ansprüche 1 bis 8, wobei das Expressionsniveau von:
ALOX12 vermindert ist, BBC erhöht ist, BHLHB9 vermindert ist, GTPBP2 vermindert ist, HDDC3 erhöht ist, ID2 vermindert ist, LCE2B vermindert ist, LOC100287896 erhöht ist, MMP10 vermindert ist, MSANTD4 vermindert ist, NFASC vermindert ist, NFIC vermindert ist, PDPN erhöht ist, RCHY1 erhöht ist, RPP38 vermindert ist, RUNX3 erhöht ist, TAF6L erhöht ist, TFAP2B vermindert ist, ZNF48 erhöht ist und ZNF496 erhöht ist, wenn ein rezidiver Tumor mit einer nicht rezidiven Probe verglichen wird.

## Revendications

1. Méthode permettant de prédire le risque de récidive, de métastases, ou les deux, chez un patient atteint d'une tumeur à carcinome cutané à cellules squameuses (cSCC), la méthode comprenant :
(a) l'isolement de l'ARNm d'un échantillon de tumeur à cSCC qui a été prélevé chez le patient ;
(b) la détermination du niveau d'expression de ALOX12, BBC, BHLHB9, GTPBP2, HDDC3, ID2, LCE2B, LOC100287896, MMP10, MSANTD4, NFASC, NFIC, PDPN, RCHY1, RPP38, RUNX3, TAF6L, TFAP2B, ZNF48 et ZNF496 ;
(c) la détermination d'une indication quant à savoir si la tumeur à cSCC présente un risque faible à élevé de récidive locale, de métastases à distance, ou les deux, sur la base des niveaux d'expression de ALOX12, BBC, BHLHB9, GTPBP2, HDDC3, ID2, LCE2B, LOC100287896, MMP10, MSANTD4, NFASC, NFIC, PDPN, RCHY1, RPP38, RUNX3, TAF6L, TFAP2B, ZNF48 et ZNF496.

2. Méthode selon la revendication 1, comprenant en outre la comparaison des niveaux d'expression de ALOX12, BBC, BHLHB9, GTPBP2, HDDC3, ID2, LCE2B, LOC100287896, MMP10, MSANTD4, NFASC, NFIC, PDPN, RCHY1, RPP38, RUNX3, TAF6L, TFAP2B, ZNF48 et ZNF49 de l'échantillon de tumeur à cSCC aux niveaux d'expression de ALOX12, BBC, BHLHB9, GTPBP2, HDDC3, ID2, LCE2B, LOC100287896, MMP10, MSANTD4, NFASC, NFIC, PDPN, RCHY1, RPP38, RUNX3, TAF6L, TFAP2B, ZNF48 et ZNF49 à partir d'un ensemble pour apprentissage prédictif de manière à générer un score de probabilité du risque de récidive locale, de métastases à distance, ou les deux.

3. Méthode selon la revendication 1 ou 2, ledit niveau d'expression de chaque gène dans l'ensemble de gènes étant déterminé par transcription inverse de l'ARNm isolé en ADNc et par mesure d'un niveau de fluorescence pour chaque gène dans l'ensemble de gènes par un système de détection de séquence d'acide nucléique suite à une réaction en chaîne par polymérase en temps réel (RT-PCR).

4. Méthode selon l'une quelconque des revendications 1 à 3, ledit échantillon de tumeur à cSCC étant obtenu à partir d'un échantillon fixé à la formaline et inclus dans la paraffine.

5. Méthode selon l'une quelconque des revendications 2 à 4, ledit score de probabilité de récidive locale, de métastases à distance ou les deux, étant compris entre 0 et 1, une valeur de 1 indiquant une probabilité plus élevée de récidive locale, de métastases à distance ou les deux, qu'une valeur de 0, et ledit score de probabilité de 0 à 1 reflétant la corrélation du niveau d'expression de l'échantillon de tumeur à cSCC avec l'ensemble pour apprentissage utilisé pour prédire le résultat de risque.

6. Méthode selon l'une quelconque des revendications 2 à 5, ledit score de probabilité étant une analyse bimodale à deux classes, un patient comportant une valeur comprise entre 0 et 0,499 étant désigné comme étant de classe 1 (risque faible) et un patient comportant une valeur comprise entre 0,500 et 1,00 étant désigné comme étant de classe 2 (risque élevé), et ledit score de probabilité de 0 à 1 reflétant la corrélation du niveau d'expression de l'échantillon de tumeur à cSCC avec l'ensemble pour apprentissage utilisé pour prédire le résultat de risque.

7. Méthode selon l'une quelconque des revendications 2 à 6, ledit score de probabilité étant une analyse trimodale à trois classes, les patients étant désignés comme étant de classe A (risque faible), de classe B (risque intermédiaire) ou de classe C (risque élevé).

8. Méthode selon l'une quelconque des revendications 2 à 7, comprenant en outre l'identification de la tumeur à cSCC comportant un risque élevé de récidive locale, de métastases à distance, ou les deux, sur la base du score de probabilité, et l'administration au patient d'un traitement tumoral agressif.

9. Méthode selon l'une quelconque des revendications 1 à 8, ledit niveau d'expression de :
ALOX12 étant diminué, BBC étant accru, BHLHB9 étant diminué, GTPBP2 étant diminué, HDDC3 étant accru, ID2 étant diminué, LCE2B étant diminué, LOC100287896 étant accru, MMP10 étant diminué, MSANTD4 étant diminué, NFASC étant diminué, NFIC étant diminué, PDPN étant accru, RCHY1 étant accru, RPP38 étant diminué, RUNX3 étant accru, TAF6L étant accru, TFAP2B étant diminué, ZNF48 étant accru et ZNF496 étant accru lors de la comparaison d'une tumeur récurrente à un échantillon non récurrent.
